# EUROPEAN PATENT SPECIFICATION

(11) **EP 4 453 560 B1**
(45) Date of publication and mention of the grant of the patent: **17.06.2026**
(21) Application number: 22839793.1
(22) Date of filing: 19.12.2022
(51) Int. Cl.: G01N 30/86, A61K 39/00, G01N 30/88, A61K 39/39, C07K 16/00

(54) **METHOD FOR DETERMINING THE DEGREE OF OXIDATIVE DEGRADATION OF POLYSORBATE 20 IN AQUEOUS FORMULATIONS**
VERFAHREN ZUR BESTIMMUNG DES GRADS DES OXIDATIVEN ABBAUS VON POLYSORBAT 20 IN WÄSSRIGEN FORMULIERUNGEN
PROCÉDÉ PERMETTANT DE DÉTERMINER LE DEGRÉ DE DÉGRADATION OXYDATIVE DE POLYSORBATE 20 DANS DES FORMULATIONS AQUEUSES

(30) Priority: 20.12.2021 EP 21215813
(43) Date of publication of application: 30.10.2024
(73) Proprietor: BOEHRINGER INGELHEIM INTERNATIONAL GMBH, 55216 Ingelheim am Rhein (DE)
(72) Inventor: ZEUN, Aline, 55216 Ingelheim am Rhein (DE); HALBACH, Felix, 55216 Ingelheim am Rhein (DE)
(74) Representative: Höfer, Friederike
(86) International application number: PCT/EP2022/086583
(87) International publication number: WO 2023/117858

(56) References cited:
- WO-A1-2017/117311
- WO-A1-2020/174064
- ZHANG LIN ET AL: "Degradation Mechanisms of Polysorbate 20 Differentiated by18O-labeling and Mass Spectrometry", PHARMACEUTICAL RESEARCH, SPRINGER US, NEW YORK, vol. 34, no. 1, 13 October 2016 (2016-10-13), pages 84 - 100, XP036119652, ISSN: 0724-8741, [retrieved on 20161013], DOI: 10.1007/S11095-016-2041-Y
- DAHOTRE SANKET ET AL: "Novel markers to track oxidative polysorbate degradation in pharmaceutical formulations", JOURNAL OF PHARMACEUTICAL AND BIOMEDICAL ANALYSIS, ELSEVIER B.V, AMSTERDAM, NL, vol. 157, 21 May 2018 (2018-05-21), pages 201 - 207, XP085408219, ISSN: 0731-7085, DOI: 10.1016/J.JPBA.2018.05.031
- OLEG V. BORISOV ET AL: "Oxidative Degradation of Polysorbate Surfactants Studied by Liquid Chromatography and Mass Spectrometry", JOURNAL OF PHARMACEUTICAL SCIENCES, vol. 104, no. 3, 1 March 2015 (2015-03-01), US, pages 1005 - 1018, XP055585465, ISSN: 0022-3549, DOI: 10.1002/jps.24314

## Description

### BACKGROUND OF THE INVENTION

### TECHNICAL FIELD

The invention relates to a method for determining the degree of oxidative degradation of polysorbate 20 in aqueous formulations.

### STATE OF THE ART

Polysorbate (PS) constitutes a family of non-ionic surfactants, which are widely used as wetting agents or as emulsifiers, for example in cosmetics, medicines, foodstuffs, cleaning agents and detergents. Due to their biocompatibility and low toxicity polysorbates are the most widely used surfactants for biopharmaceutical formulations and serve as excipients in biopharmaceutical formulations to solubilise substances, such as proteins, in water that are not water-soluble per se. In particular, they stabilise proteins against interfacial stress, and prevent interface induced aggregation or surface adsorption. In this way, they help to stabilise ingredients in an aqueous formulation, for example, proteins, and thus maintain the ingredients' activity and efficacy.

Polysorbate is derived of a sorbitan or isosorbid core structure which is linked to poly(oxy)ethylene (POE) chains of variable length. These may in turn be esterified by fatty acids.

Exemplary representatives of polysorbates are
polysorbate 20 (PS20) or polyoxyethylene (20) sorbitan monolaurate;
polysorbate 40 (PS40) or polyoxyethylene (20) sorbitan monopalmitate;
polysorbate 60 (PS60) or polyoxyethylene (20) sorbitan monostearate; and
polysorbate 80 (PS80) or polyoxyethylene (20) sorbitan monooleate.

The numbers 20, 40, 60 and 80 which follow the term "polysorbate" stand for the type of fatty acid associated with the polyoxyethylene sorbitan residue of the molecule, i.e. monolaurate is indicated by 20, monopalmitate is indicated by 40, monostearate is indicated by 60, and monooleate is indicated by 80. The number 20 following the term "polyoxyethylene" refers to the total number of oxyethylene -(CH₂CH₂O)- groups present in the molecule. Commercially available polysorbates usually consist of a mixture of structurally related molecules such as nonesterified sorbitan and/or isosorbitan-polyethylene glycol (PEG) species, mono-, di-, tri-, tetra-esters, fatty acids, and the like.

Two main types of polysorbate are most relevant for biopharmaceutical formulations, namely polysorbate 20 (PS0) and polysorbate 80 (PS80), which mainly differ in the distribution of fatty acids and poly(oxy)ethylene-chain elements. Polysorbate 40 and 60 are more commonly used in the food or cosmetics industry.

But polysorbates exhibit a number of degradation reactions, whereby numerous degradation products are formed and functional properties are lost, potentially leading to the instability of the aqueous formulations used. Therefore, the degradation of polysorbate leads to particle formation in the aqueous formulations, which can be a major quality concern and potential risk factor.

For the degradation of polysorbate, for example observed in biopharmaceutical products, two main degradation pathways exist: hydrolytic degradation, which is typically catalyzed by host cell impurities and represents an ester hydrolysis, which leads to liberation of free fatty acid, and an oxidative degradation, which is initiated by free radicals, which is also referred to as autoxidation. As is known in general, autoxidation can occur anywhere within a molecule as long as the kinetic characteristics of H-abstraction at a given site are favorable. In this context, it must be taken into account that for complex molecules, such as polysorbate 20, there can generally be a very large number of degradation products that can be detected with a number of different analytical techniques. In [1, 2], for example, about 60 different species are assumed. Although autoxidation of unsaturated fatty acids, in particular oleic acid, has been extensively studied for decades because of its importance in the oxidation of lipids in foods and oils, less is known about the actual degradation pathways and stabilities of the individual components of polysorbates under oxidative stress.

Oxidative degradation of both polysorbate 20 and polysorbate 80 has been already described in literature. These references aim to describe the oxidative degradation mechanism which either occurs along the unsaturated fatty acid structures wherein a double bond is present [1] or within the poly(oxy)ethylene chain structure [2] as both elements are susceptible to free radical induced fragmentation.

For polysorbate 80, the first pathway prevails due to the presence of oleic acid with its double bond. Consequently, specific individual components arising due to the oxidative degradation have been identified that represent distinct substructures which emerge during degradation and can be detected by LC-MS (Liquid Chromatography-Mass Spectrometry) analytics. Such individual components are also referred to as oxidation markers. For example, in [1] the major degradation products of polysorbate 20 and polysorbate 80 have been evaluated upon oxidative stress induced by 2,2'-azobis-2-methyl-propanimidamide, dihydrochloride (AAPH). AAPH is a water-soluble azo compound used as a model oxidizer and a free radical generator. The oxidative markers of polysorbate 80 found include several structures such as poly(oxy)ethylene sorbitan esterified with hydroperoxy-oleate (hydroperoxy-C18:1), keto-oleate (keto-C18:1), hydroxyl-oleate (hydroxyl-C18:1), epoxy-stearate (epoxy-C18:0), and oxo-nonanoate (oxo-C9:0), which have been described including the mass-to-charge ratios (m/z) of the respective ions used in mass spectrometry that allow their analysis with LC-MS based detection.

For polysorbate 20, due to the lack of unsaturated fatty acids, oxidative degradation of polysorbate mainly proceeds via fragmentation of the ether bond within the poly(oxy)ethylene structure [2] which does not necessarily lead to the emergence of novel structures that may be used as marker structures. Therefore, current literature has not yet described any oxidative marker structures for liquid chromatography-mass spectrometry (LC-MS) based analytics that target the poly(oxy)ethylene sub-structure region.

According to Borisov et al. [1] it is proposed that poly(oxy)ethylene-esters with 4 to 5 oxy-ethylene (OE) units are byproducts of oxidative degradation. Kishore et al. [2] describe poly(oxy)ethylene-esters with 1 to 6 oxy-ethylene units. Zhang et al. [3] describe the formation of poly(oxy)ethylene-esters (POE-laurates) with 1 to 8 ethylene oxide (EO) units, a mixture wherein POE-laurate with 6EO units is predominant, but in a situation where L-Histidine and polysorbate 20 are present at the same time and under forced oxidation conditions, whereby the solution was additionally saturated with O₂ gas. However, none of these studies provide chemical structures and corresponding mass-to-charge ratios of the ions (m/z ratios) which could allow detection and analysis via liquid chromatography-mass spectrometry (LC-MS) based methods. In the above-mentioned literature it is demonstrated that all poly(oxy)ethylene-laurates, for example those having 1 to 8 oxy-ethylene units, arise from oxidative stress, starting from polysorbate 20 used as the stressed material. This is typically not transferable to aqueous formulations under ambient conditions, and is particularly not applicable to biopharmaceutical formulations. Indeed, this does not represent a valid approach for practice.

More importantly, the degradation products were artificially produced using a substance for generating free radicals that is far removed from the real circumstances and conditions of usual aqueous formulations. Furthermore, the realities within, for example, native biopharmaceutical formulations e.g. under routine stability conditions were not taken into account. Furthermore, the degradation products were not linked to the oxidative degradation of polysorbate without the use of artificial radical initiators. In addition, none of the degradation products have been linked to mass-to-charge ratios of the ions (m/z ratios) that could allow their monitoring via liquid chromatography-mass spectrometry (LC-MS) based methods.

Besides liquid chromatography-mass spectrometry (LC-MS) based label-free analytics, the oxidative degradation of polysorbate has been investigated using other techniques. For example, the use of 2,4-dinitrophenylhydrazine has been described for derivatization of free fatty acid esters that arose as byproducts of oxidative stress applied to polysorbate 20 [4] as has the use of charged aerosol detection coupled to reversed phase chromatography [5]. However these techniques do not clarify individual degradation species of polysorbate under oxidative stress.

However, degradation of polysorbate is a major challenge particularly in biopharmaceutical development and may pose serious risks to successful product development by e.g. deteriorating product quality or raising safety issues. Therefore, understanding the causes and processes of polysorbate degradation is crucial to set up suitable remedial strategies. According to the current state of the art, a differentiation between hydrolytic and oxidative degradation of polysorbate by direct and unambiguous analytical tools is not possible, and specifically no substances to identify the presence of an oxidative degeneration of polysorbate 20 are known, i.e. no marker substances are known to be derived from the oxidative degradation of polysorbate 20, which is widely used as a surfactant in aqueous formulations. Moreover, to date no analytical methods have been described to detect and quantify such marker substances within aqueous formulations, particularly in biopharmaceutical formulations.

Furthermore, the liquid chromatography-mass spectrometry (LC-MS) method that has been disclosed, for example in [1], is not intended as a method for absolute quantification of fatty acid esters in polysorbate, only a semiquantitative determination is possible using that method.

Moreover, a method to study the mechanisms of polysorbate (PS) degradation is disclosed in [13]. Ultra-performance liquid chromatography-mass spectrometry (UPLC-MS) was used to characterise polysorbate 20 and its degradation products by measuring ¹⁸O-incorporation, the two main so-called decomposition products being free lauric acid and polyoxyethylene (POE) laurate.

According to WO 2020/1744064 A1 [14], there is provided a process for quantifying at least one polysorbate derivative in a sample, the process comprising:
- the step of performing LC-MS analysis of the sample based on the signal from the dioxolanylium ion;
- the step of performing an internal calibration with an internal standard of the polysorbate.

The internal standard refers to a chemical compound that is added in a constant amount to the samples, blank and calibration standards in a chemical analysis. It is a compound that is generally very similar, but not identical, to the polysorbate in the samples. In one embodiment, the internal standard (IS) may be selected from compounds having a polyethylene glycol chain esterified with one or more carboxylic acids such that it has similar physicochemical properties to the polysorbate.

In WO 2017/117311 A1 [15], a method for reducing polysorbate degradation in an aqueous formulation comprising a polysorbate is described, the method comprising adding a cyclodextrin to the formulation, wherein the resulting weight ratio of cyclodextrin to polysorbate is greater than about 37.5:1.

In fact, the patent and academic literature in whole is completely silent about the use of specific marker substances, particularly label-free oxidation marker species, that would be specific for polysorbate 20 and exclusively linked to naturally occurring oxidative polysorbate degradation. Moreover, up to now, no oxidation byproduct has been identified as an established marker to track polysorbate 20 oxidation in aqueous formulations containing polysorbate 20, particularly in biopharmaceutical formulations including biological APIs (Active Pharmaceutical Ingredient(s)) and usual excipients.

It is therefore an object of the present invention to overcome at least these deficiencies of the prior art and to provide a method in which a marker substance, particularly a label-free oxidation marker species that is specific for polysorbate 20, may be identified and determined, which is exclusively linked to the oxidative degradation of polysorbate 20. This method should allow one to monitor and quantify the oxidation marker substance in aqueous formulations.

### GENERAL DESCRIPTION OF THE INVENTION

Surprisingly, it was found that a concrete marker substance, namely polyethylene glycol-6-laurate (PEG-6-laurate), is linked to the oxidative degradation route of polysorbate 20, because this marker substance is formed during oxidative degradation of polysorbate 20. This marker substance allows one to determine the degree of oxidative degradation of polysorbate 20 in aqueous formulations by an analytical method, since the relative level of said marker substance indicates that an oxidative degradation has taken place.

Therefore, in a first aspect, the present invention provides a (first) method (in a first alternative) for determining the degree of oxidative degradation of polysorbate 20 in an aqueous formulation containing polysorbate 20, said method comprising the following steps:

| | |
|---|---|
| step 1 | providing a first sample of the aqueous formulation to be examined, which serves as a reference sample; |
| step 2 | providing a second sample and optionally further samples of the aqueous formulation to be examined; |
| step 3 | using a liquid chromatography-mass spectrometry based method for separating polyethylene glycol-6-laurate and determining the peak area of polyethylene glycol-6-laurate, the oxidation marker for the oxidative degradation of polysorbate 20, from the reference sample of step 1, and from the second sample and each optional further sample of step 2, respectively, and determining the relative peak area of polyethylene glycol-6-laurate based on the peak area of polyethylene glycol-6-laurate in the second and each optional further sample in relation to the peak area of polyethylene glycol-6-laurate in the reference sample, the relative peak area being proportional to the degree of oxidative degradation of polysorbate 20; and |
| step 4 | optionally quantifying the amount of polyethylene glycol-6-laurate in the second sample and each optional further sample based on the relative peak area of the respective sample. |

According to an embodiment the relative peak area of polyethylene glycol-6-laurate for the second sample and each optional further sample in step 3 is determined by
- calculating the difference of the peak areas obtained, wherein the peak area of polyethylene glycol-6-laurate in the reference sample is subtracted, respectively, from the peak area of polyethylene glycol-6-laurate in the second sample and from the peak area of polyethylene glycol-6-laurate in each of the optional further sample(s);
   or
- normalising the peak areas obtained, wherein the peak area of polyethylene glycol-6-laurate in the reference sample is set equal to 100% and the peak area of polyethylene glycol-6-laurate in the second sample and in each of the optional further sample(s) is calculated respectively based thereon.

Thus, the method of the present invention provides the identification of polyethylene glycol-6-laurate as a byproduct of oxidative degradation of polysorbate 20 in aqueous formulations containing polysorbate 20 and makes it possible to separate, detect and determine this marker substance relative to a reference by a liquid chromatography-mass spectrometry based method (LC-MS based method) and to optionally quantify this marker substance in aqueous formulations by a specific analytical method. Since the peak area (integral) is known to be proportional to the concentration of an analyte, the relative peak area makes it possible to correlate the relative peak area of polyethylene glycol-6-laurate with the degree of oxidative degradation of polysorbate 20.

In the first alternative of the (first) method given above, the first sample of the aqueous formulation to be tested is taken as a reference sample.

In an alternative embodiment, an externally produced or provided reference sample is used as a reference, i.e. an external reference sample is provided independently.

Therefore, according to a second aspect, the present invention provides a (first) method (in a second alternative) for determining the degree of oxidative degradation of polysorbate 20 in an aqueous formulation containing polysorbate 20, said method comprising the following steps:

| | |
|---|---|
| step 1' | providing a reference sample which is an aqueous solution containing polysorbate 20, the polysorbate 20 content being the same as in the aqueous formulation to be examined; |
| step 2' | providing a first sample and optionally further samples of the aqueous formulation to be examined; |
| step 3' | using a liquid chromatography-mass spectrometry based method for separating polyethylene glycol-6-laurate and determining the peak area of polyethylene glycol-6-laurate, the oxidation marker for the oxidative degradation of polysorbate 20, from the reference sample of step 1', and from the first sample and each optional further sample of step 2', respectively, and determining the relative peak area of polyethylene glycol-6-laurate based on the peak area of polyethylene glycol-6-laurate in the first and each optional further sample in relation to the peak |
| | area of polyethylene glycol-6-laurate in the reference sample, the relative peak area being proportional to the degree of oxidative degradation of polysorbate 20; and |
| step 4' | optionally quantifying the amount of polyethylene glycol-6-laurate in the first sample and each optional further sample based on the relative peak area of the respective sample. |

According to an embodiment the relative peak area of polyethylene glycol-6-laurate for the first and each optional further sample in step 3' is determined by
- calculating, the difference of the peak areas obtained, wherein the peak area of polyethylene glycol-6-laurate in the reference sample is subtracted, respectively, from the peak area of polyethylene glycol-6-laurate in the first sample and from the peak area of polyethylene glycol-6-laurate in each of the optional further sample(s);
   or
- normalising the peak areas obtained, wherein the peak area of polyethylene glycol-6-laurate in the reference sample is set equal to 100% and the peak area of polyethylene glycol-6-laurate in the first sample and in each of the optional further sample(s) is calculated respectively based thereon.

In an embodiment of the invention, in step 2 or step 2', several samples of the aqueous formulation containing polysorbate 20 are taken at successive times T1, T2, T3.... so that not only a random sample but the course of possible oxidative degradation of polysorbate 20 in the aqueous formulation over time can be determined. For example, in step 2, two, three or more further samples of the aqueous formulation containing polysorbate 20 can be taken at successive time intervals T1, T2, T3.... which may be of the same or different lengths.

In the first method given above (first and second alternative), one aqueous formulation is tested for the oxidative degradation of polysorbate 20. However, several aqueous formulations can also be examined. Therefore, in a further embodiment of the invention it is provided a (second) method for determining the degree of oxidative degradation of polysorbate 20 in a number of aqueous formulations, for example, two, three, four or more aqueous formulations, that respectively contain polysorbate 20, in particular each aqueous formulation containing the same amount of polysorbate 20, by the following steps:

| | |
|---|---|
| step 1a | providing a sample of each aqueous formulation to be examined; |
| step 2a | using a liquid chromatography and mass spectrometry based method for separating polyethylene glycol-6-laurate and determining the peak area of polyethylene glycol-6-laurate, the oxidation marker for the oxidative degradation of polysorbate 20, from each sample of step 1a, and |
| | comparing the peak areas of the samples with each other, wherein the difference in peak area (the relative peak area) of the samples is proportional to the degree of oxidative degradation of polysorbate 20 in each respective sample; and |
| step 3a | optionally quantifying the amount of polyethylene glycol-6-laurate in each sample based on the obtained peak area of the respective sample. |

In this (second) method of the invention, the peak areas can be directly compared with each other, or it is also possible to determine the relative peak area using a reference and then compare the sample(s) to the reference. The reference can be one of the samples taken, whereby the choice of the sample that can be used as a reference depends on the individual case. The reference can also be an external reference sample, as already described in connection with the first methods.

In the (second) method the relative peak area of polyethylene glycol-6-laurate in each sample may be determined in relation to a reference sample and this procedure is then carried out by
- calculating the difference of the peak areas obtained, wherein the peak area of polyethylene glycol-6-laurate in a reference sample is subtracted, respectively, from the peak area of polyethylene glycol-6-laurate in each sample of step 2a;
   or
- normalising the peak areas obtained, wherein the peak area of polyethylene glycol-6-laurate in a reference sample is set equal to 100% and the peak area of polyethylene glycol-6-laurate in each sample of step 2a is calculated based thereon.

The separation of polyethylene glycol-6-laurate identified to be an oxidative marker substance of the oxidative degradation of polysorbate 20 is achieved by the liquid chromatography as part of the liquid chromatography-mass spectrometry based method by using its hydrophobic property. In the subsequent mass spectrometry procedure, the molecules to be examined are transferred into the gas phase and ionised. The molecules can be fragmented in this method. The detection and determination of the relative level of polyethylene glycol-6-laurate in step 3, step 3' or step 2a is thus performed using the liquid chromatography-mass spectrometry based method. The optional quantification in step 4, step 4' or step 3a may be, for example, performed with an external calibration using standard solutions of polyethylene glycol-6-laurate.

According to an embodiment of the liquid chromatography-mass spectrometry based method the liquid chromatography is used as separation chromatography, particularly in the form of column chromatography, more particularly used as reversed-phase chromatography.

In a further embodiment the liquid chromatography as part of the liquid chromatography-mass spectrometry based method is a high performance liquid chromatography (HPLC). Also an ultra performance liquid chromatography (UPLC) may be used.

According to an embodiment of the liquid chromatography-mass spectrometry based method in the liquid chromatography a gradient elution is used.

In another embodiment a polar solvent is added to the polysorbate-containing sample to be investigated before liquid chromatography is carried out, the polar solvent being in particular the solvent used in liquid chromatography.

In a further embodiment the mass spectrometry as part of the liquid chromatography-mass spectrometry based method is selected to be a high resolution-mass spectrometry (HR-MS). High-resolution mass spectrometry may be used in particular for structure elucidation and is advantageous for this purpose.

According to an embodiment for the data evaluation of the liquid chromatography-mass spectrometry based method of the invention extracted ion chromatograms are generated from a mass-to-charge ratio which comprises a range of about 487 amu, in particular comprising or consisting of a range of 486.5 to 487.5 amu.

An additional optional step 5, which can follow step 4 or step 3, or an additional optional step 5', which can follow step 4' or step 3', or an additional optional step 4a which can follow step 3a or step 2a, comprises estimating to what extent polysorbate 20 has already been oxidatively degraded in the sample based on the inversely proportional relationship between the polyethylene glycol-6-laurate level and the polysorbate 20 level. It was found that a decreasing content of polysorbate 20 in the sample and thus in the aqueous formulation clearly correlates with an increasing relative level of the oxidative marker substance polyethylene glycol-6-laurate, whereby polysorbate 20 is increasingly degraded.

According to a further embodiment of the invention the aqueous formulation contains one or more proteins. The protein(s) may be selected from a monoclonal antibody, polyclonal antibody, monospecific antibody, multispecific antibody, single chain antibodies or antibody fragments thereof, such as, Fv, scFv, Fab, Fab', scFab, F(ab')2, Fab2, Fc and Fc'-fragments, heavy and light immunoglobulin chains and their constant, variable or hypervariable region as well as Fv- and Fd-fragments, diabody, triabody, scFv-Fc, minibody, or single domain antibody or a mixture thereof. The protein(s) may be selected from therapeutic protein(s), which are used for the prevention or treatment of a disease or a disorder.

According to another embodiment the aqueous formulation may be an aqueous pharmaceutical formulation, particularly an aqueous biopharmaceutical formulation, which contains polysorbate 20. For example, the aqueous biopharmaceutical formulation may contain pharmaceutically acceptable polysorbate 20, optionally API(s) such as one or more proteins, which are used for the prevention or treatment of a disease or a disorder, and optionally pharmaceutically acceptable excipient(s).

The polysorbate 20 present may be intended for pharmaceutical use, particularly for use in drugs. Particularly, the polysorbate 20 is pharmaceutically acceptable and may have pharma grade quality such as Ph. Eur./USP/China Pharmacopoeia grade quality. For example, the polysorbate 20 may be suitable for parenteral administration. Exemplarily mentioned are the following commercial products of polysorbate 20: Super Refined^{™}PS20, Tween^{™}20, Tween^{™}20 HP, Tween^{™}20 pharma grade or PS20 HP, particularly available from Croda, Edison, NJ, USA or Croda Europe Ltd. or PS20 China grade, particularly available from Nanjing Well Pharmaceutical Co., Ltd.

The invention also relates to a process (third method) of producing a recombinant protein, said process comprising the following steps:

| | |
|---|---|
| step a) | cultivating in a cell culture an eukaryotic cell expressing a recombinant protein; |
| step b) | harvesting the recombinant protein, |
| step c) | purifying the recombinant protein using an aqueous formulation; and |
| step d) | using polysorbate 20, formulating the recombinant protein into a pharmaceutically acceptable aqueous formulation suitable for administration; and |
| step e) | obtaining at least one sample of the pharmaceutically acceptable aqueous formulation of step d); |

wherein the process (third method) additionally comprises performing a (first or second) method for determining the degree of oxidative degradation of polysorbate 20 as disclosed in the present invention with the sample obtained in step e).

According to a further embodiment, the process (third method) for producing a recombinant protein comprises obtaining at least one sample containing the recombinant protein and polysorbate 20 in step e) as described above, wherein the sample from step d) is a drug substance sample or a drug product sample.

The invention is also directed to the use of polyethylene glycol-6-laurate as oxidation marker in a method for determining the degree of the oxidative degradation of polysorbate 20 in an aqueous formulation or a number of aqueous formulations that respectively contain polysorbate 20, whereby the method is the (first or second) method of the present invention as disclosed herein.

According to the present invention the (first or second) method as disclosed herein may be used as a stability testing during the development, manufacture, shelf life, or storage period of an aqueous formulation or a number of aqueous formulations that respectively contain polysorbate 20 for determining the degree of the oxidative degradation of polysorbate 20 in the aqueous formulation(s).

### BRIEF DESCRIPTION OF THE DRAWINGS

The drawings together with the general description and detailed description serve to explain the principles of the present disclosure. It is shown:

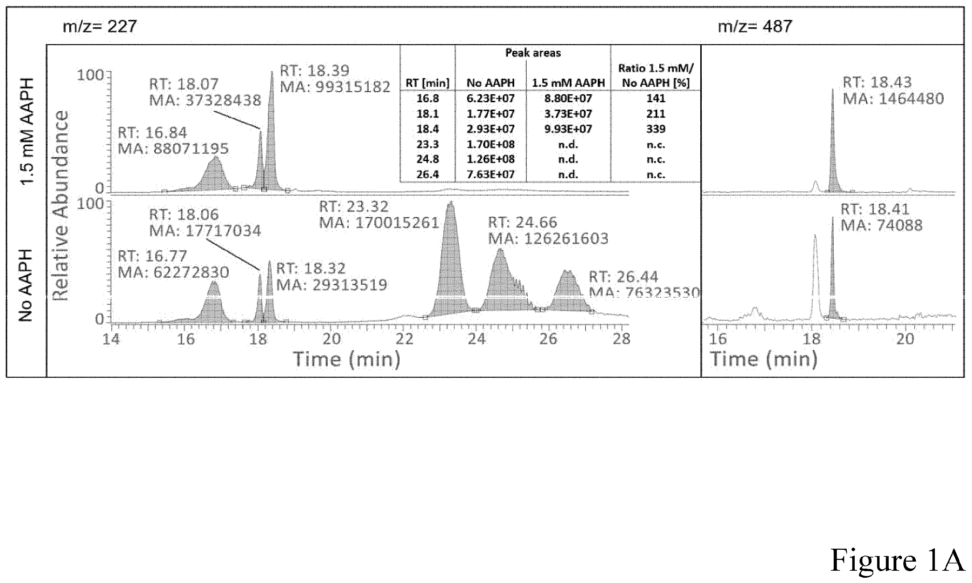

- Figure 1A: on the left side: extracts of two full-scan total ion chromatograms (TICs) of polysorbate 20 in an aqueous solution resulting from an embodiment of the liquid chromatography-mass spectrometry (LC-MS) based method of the invention, both shown at a mass-to-charge ratio m/z of about 227 amu (226.5 to 227.5 amu), in the upper spectrum oxidation catalyst AAPH is added, the lower spectrum is without oxidation catalyst AAPH; on the right side: extracts of two full-scan total ion chromatograms (TICs) of polysorbate 20 in an aqueous solution resulting from an embodiment of the liquid chromatography-mass spectrometry (LC-MS) based method of the invention, both shown at a mass-to-charge ratio m/z of about 487 amu (486.5 to 487.5 amu), in the upper spectrum oxidation catalyst AAPH is added, the lower spectrum is without oxidation catalyst AAPH;

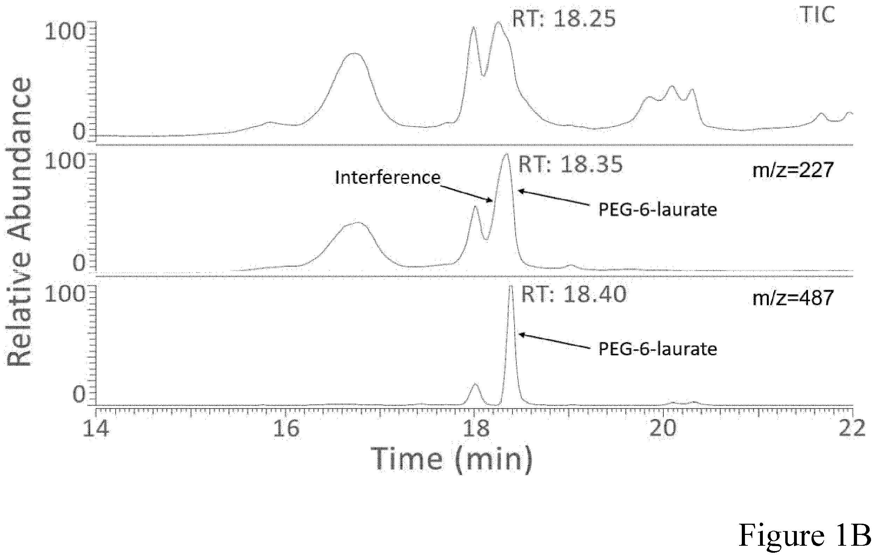

- Figure 1B: 3 ion chromatograms resulting from an embodiment of the liquid chromatography-mass spectrometry (LC-MS) based method of the invention in oxidatively stressed polysorbate 20 aqueous solution, namely the total ion chromatogram (TIC, upper panel), the extracted ion chromatogram at a mass-to-charge ratio m/z of about 227 amu (from m/z 226.5 to m/z 227.5 amu) (middle panel) and the extracted ion chromatogram at a mass-to-charge ratio m/z of about 487 amu (from m/z 486.5 to m/z 487.5 amu) (lower panel);

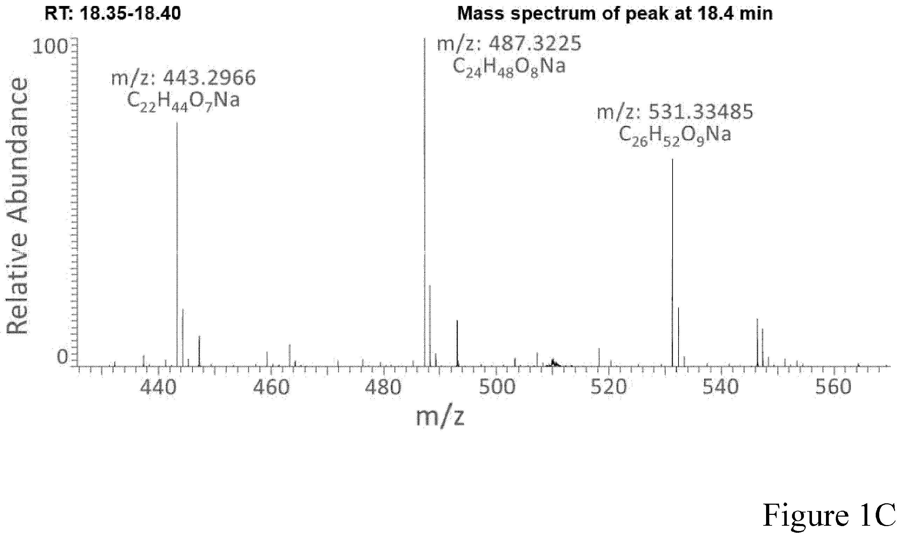

- Figure 1C: a high-resolution mass spectrum of the peak eluting at 18.4 min, resulting from an embodiment of the liquid chromatography-mass spectrometry (LC-MS) based method of the invention, shown in the chromatograms in Figures 1A and 1B;

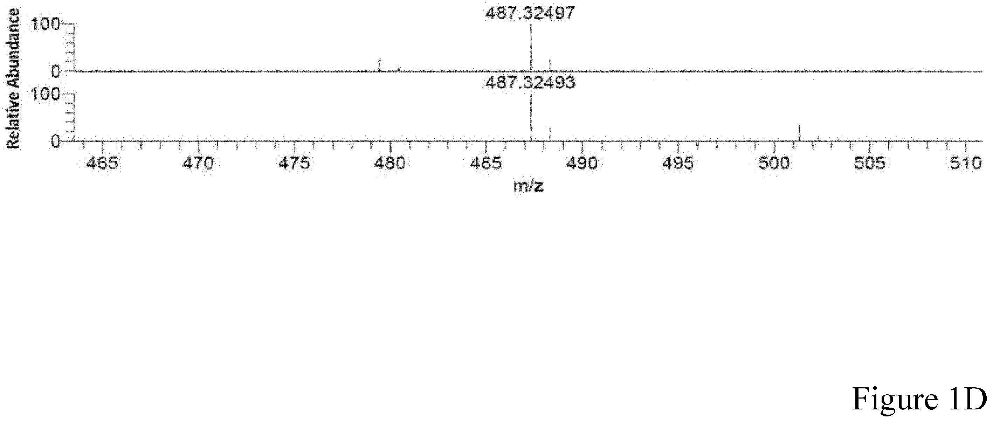

- Figure 1D: two mass spectra, one mass spectrum of commercially available polyethylene glycol-6-laurate (upper panel) and one mass spectrum of polyethylene glycol-6-laurate from AAPH-stressed polysorbate 20 (lower panel), resulting from an embodiment of the liquid chromatography-mass spectrometry (LC-MS) based method of the invention;

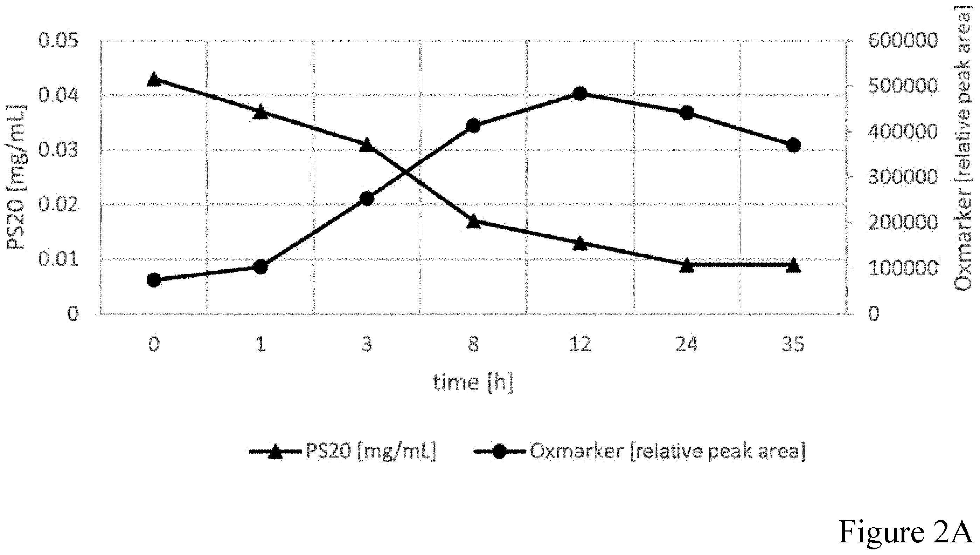

- Figure 2A: the polysorbate 20 concentration in [mg/mL] (left y axis) present in an aqueous formulation plotted against the time in [h] analysed via HPLC-CAD and the relative peak area of the oxidative marker polyethylene glycol-6-laurate (right y axis, referred to as "Oxmarker") plotted against the time in [h] resulting from an embodiment of the liquid chromatography-mass spectrometry (LC-MS) based method of the invention;

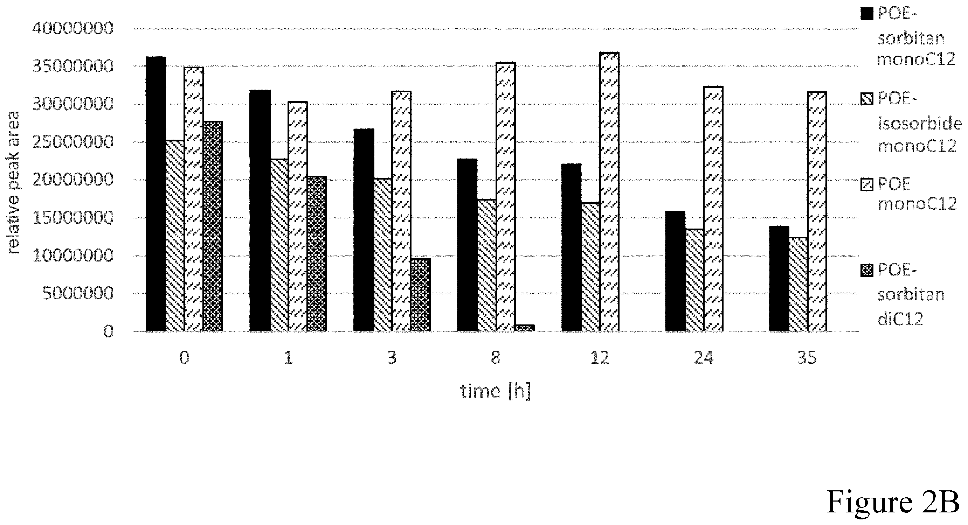

- Figure 2B: a block chart of the levels of the intact polysorbate 20 species showing the relative peak areas plotted against the time in [h] of an aqueous formulation containing polysorbate 20 using an embodiment of the liquid chromatography-mass spectrometry (LC-MS) based method of the invention;

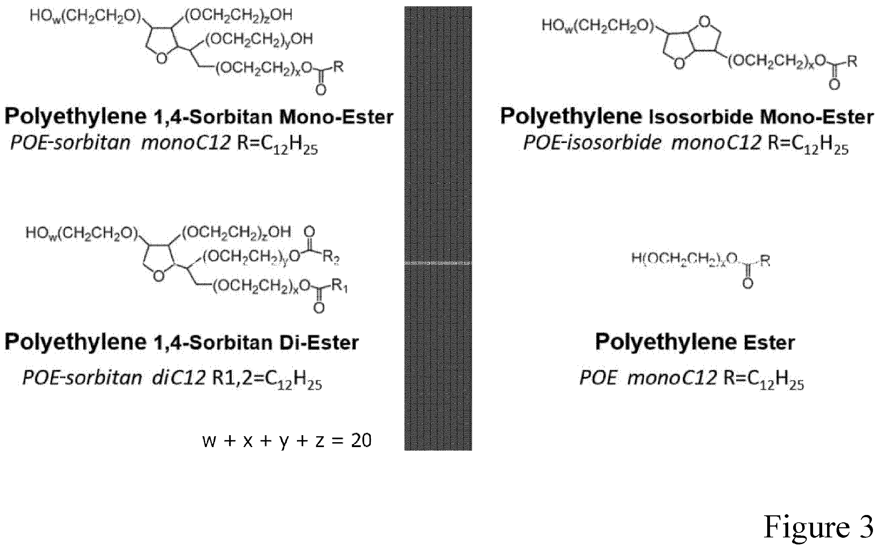

- Figure 3: the chemical structures of some intact polysorbate 20 species present in polysorbate 20, namely POE-sorbitan monoC12, POE-isosorbide monoC12, POE monoC12 and POE-sorbitan diC12;

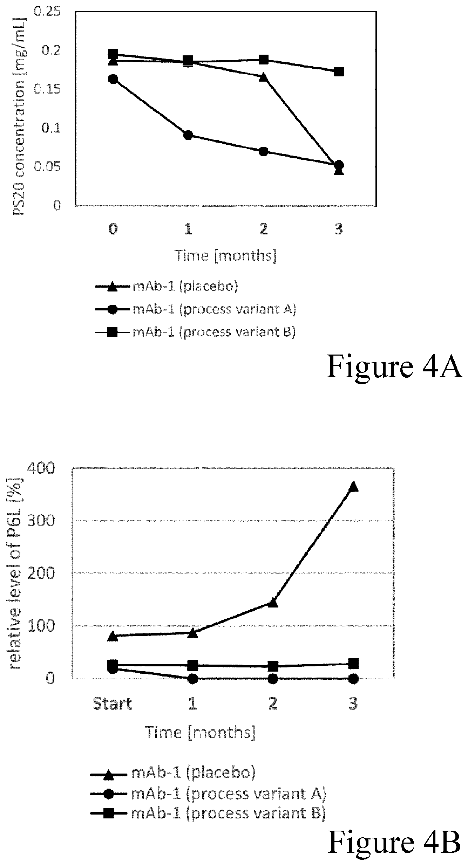

- Figure 4A: the polysorbate 20 concentration in [mg/mL] present in an aqueous formulation comprising a monoclonal antibody in different variants plotted against the time in [months] as determined by HPLC-CAD analysis;
- Figure 4B: the relative level of polyethylene glycol-6-laurate (P6L) oxidation marker based on the relative peak areas in [%] present in the aqueous formulation of Figure 4A plotted against the time in [months] as determined in the liquid chromatography-mass spectrometry (LC-MS) based method according to an embodiment of the invention;

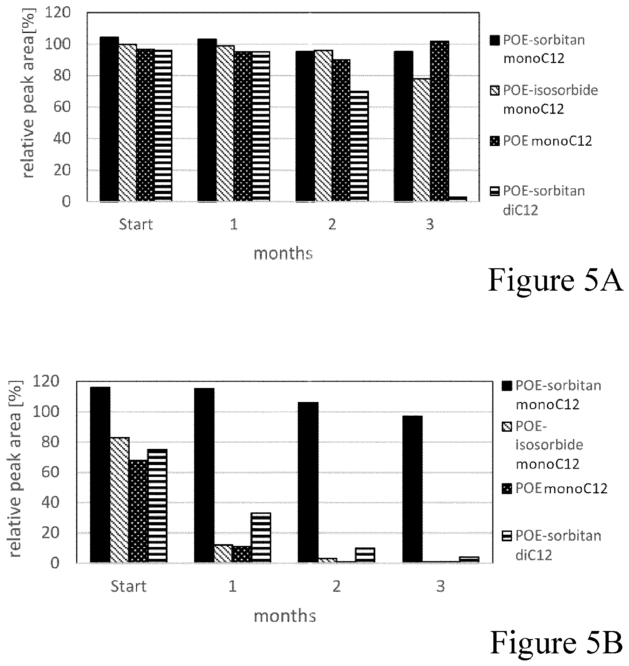

- Figure 5A: a block chart of the levels of the intact polysorbate 20 species as relative peak areas in [%] during 3 months representing a stability study of an aqueous placebo formulation (placebo to mAb-1 formulation) using an embodiment of the liquid chromatography-mass spectrometry (LC-MS) based method of the invention;
- Figure 5B: a block chart of the levels of the intact polysorbate 20 species as relative peak areas in [%] during 3 months representing a stability study of a monoclonal antibody containing aqueous formulation (mAb-1 formulation; process variant A) using an embodiment of the liquid chromatography-mass spectrometry (LC-MS) based method of the invention;

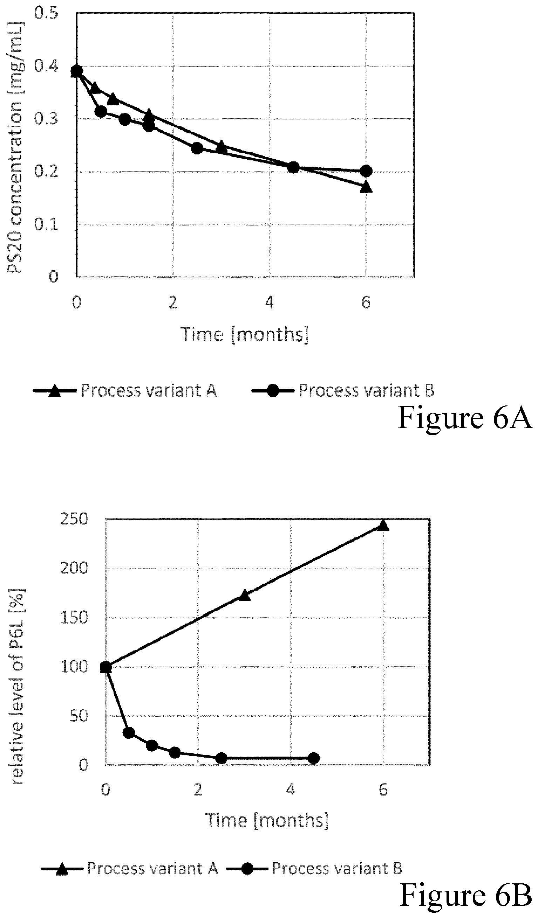

- Figure 6A: the polysorbate 20 concentration in [mg/mL] present in aqueous formulations comprising a monoclonal antibody in different variants plotted against the time in [months] as determined by HPLC-CAD analysis;
- Figure 6B: the relative level of the polyethylene glycol-6-laurate (P6L) oxidation marker based on the relative peak areas in [%] present in the aqueous formulations of Figure 6A plotted against the time in [months] as determined in the liquid chromatography-mass spectrometry (LC-MS) based method according to an embodiment of the invention;

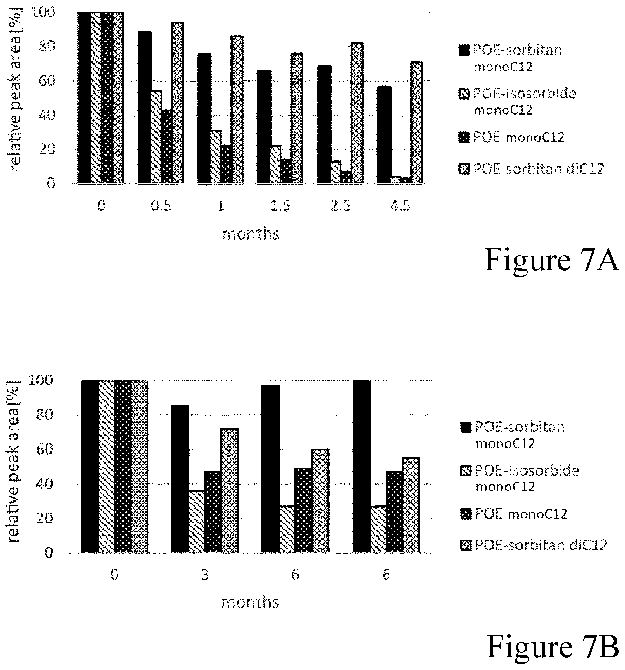

- Figure 7A: a block chart of the levels of the intact polysorbate 20 species as relative peak areas in [%] plotted against the time in [months] representing a stability study of a monoclonal antibody containing aqueous formulation (mAb-2 formulation, process variant B) using an embodiment of the liquid chromatography-mass spectrometry (LC-MS) based method of the invention;
- Figure 7B: a block chart of the levels of the intact polysorbate 20 species as relative peak areas in [%] plotted against the time in [months] representing a stability study of a monoclonal antibody containing aqueous formulation (mAb-2 formulation, process variant A) using an embodiment of the liquid chromatography-mass spectrometry (LC-MS) based method of the invention;

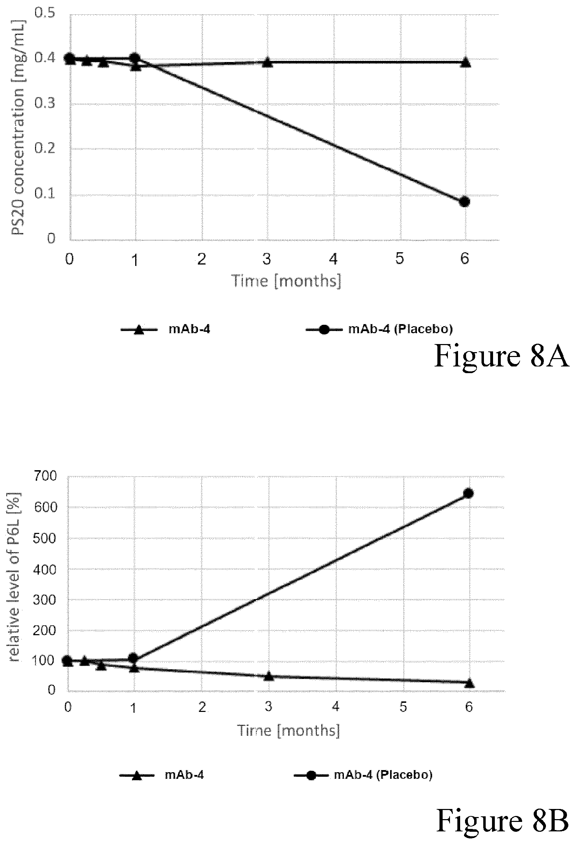

- Figure 8A: the polysorbate 20 concentration in [mg/mL] present in aqueous formulations comprising a monoclonal antibody (mAb-4 formulation) and a placebo to the monoclonal antibody (placebo to mAB-4 formulation) plotted against the time in [months] as determined by HPLC-CAD analysis;
- Figure 8B: the relative level of the polyethylene glycol-6-laurate (P6L) oxidation marker based on the relative peak areas in [%] present in the aqueous formulations of Figure 8A plotted against the time in [months] as determined in the liquid chromatography-mass spectrometry (LC-MS) based method according to an embodiment of the invention; and

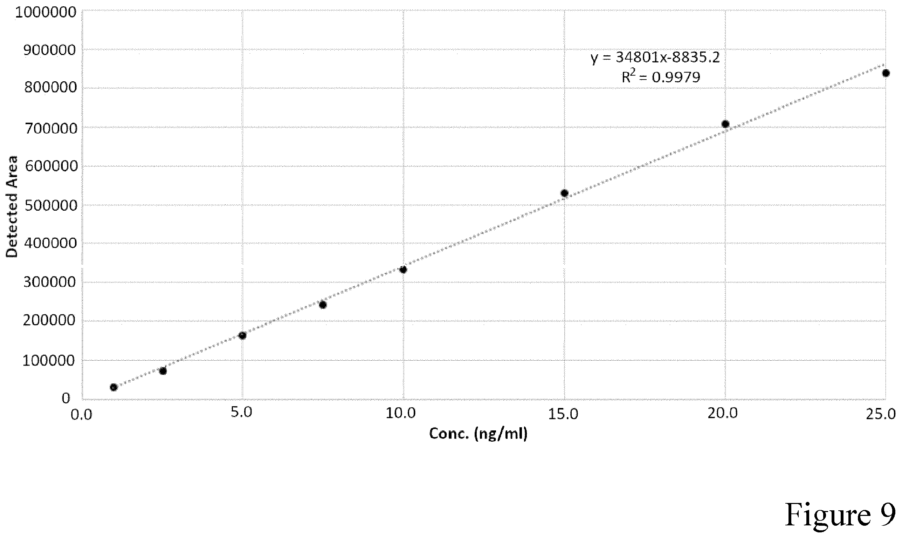

- Figure 9: standard curve for the liquid chromatography-mass spectrometry (LC-MS) based method of the present invention wherein the detected area of the liquid chromatography-mass spectrometry (LC-MS) analysis (LC-MS P6L) (y-axis) is plotted against the concentrations of standard solutions (1 - 25 ng/mL) (x-axis) of polyethylene glycol-6-laurate.

### DETAILED DESCRIPTION OF THE INVENTION

### DEFINITION OF GENERAL TERMS

Terms not specifically defined herein should be given the meanings that would be given to them by a person skilled in the art in light of the disclosure and the context.

The term "polysorbate 20" or "PS20" stands for polyoxyethylene (20) sorbitan monolaurate and is commercially available as a mixture of structurally related molecules containing mainly sorbitan polyoxyethylene fatty acid esters and also substantial amounts of polyoxyethylene, sorbitan polyoxyethylene and isosorbide polyoxyethylene [1, 2, 4]. The molecular heterogeneity of the polysorbate comes from variability within its three functional groups, that is, polydispersity with regard to the length of EO chains in the hydrophilic head, differences in chain length (carbon number) and degree of unsaturation of a fatty acid in the hydrophobic tail, and existence of species with cores consisting of sorbitol mono- and di-anhydrides (sorbitans and isosorbides, respectively). Furthermore, depending on the number of fatty acid esters per structure, polysorbates can contain mono-, di-, tri-, and tetra-esters. A summary of the compositions of various polysorbates is provided in Table 1 of [1].

According to the European Pharmacopoeia (Ph. Eur.) specification, for the content of fatty acids, the laurate ester accounts for 40-60 % of esters in polysorbate 20, the remaining esters range from C8 to C18.

For simplicity, however, it is customary to consider PS 20 as polyethoxylated (POE) sorbitan monoester of lauric acid. The theoretical structure of polysorbate 20 as shown in [6], Figure 1, is as follows:

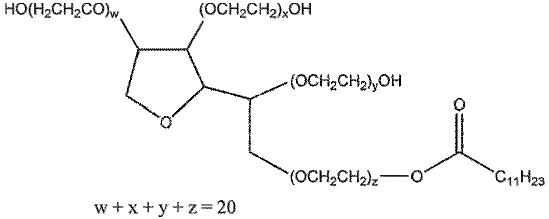

It is believed that the complex composition of the polysorbate is the reason why it represents an excellent surfactant with many applications.

The term "oxidation marker" or "oxidative marker" (also abbreviated as oxmarker) as used herein means a compound associated with the oxidative degradation of polysorbate 20, in particular a compound formed during oxidative degradation thereof. The presence of the oxidation marker at a higher level higher than in the case of an impurity therefore means that oxidative degradation of polysorbate 20 has occurred or is occurring. The oxidation marker here is polyethylene glycol-6-laurate.

The expression "oxidative degradation" of polysorbate 20 refers to degradation initiated by free radicals, which is also known as autoxidation. Oxidative degradation differs from hydrolytic degradation of polysorbate 20, which is typically catalysed by host cell impurities and is an ester hydrolysis leading to the release of free fatty acid.

The term "degree of oxidative degradation" as used in the present invention is indicative of the extent of oxidative degradation of polysorbate 20, in particular how far oxidative degradation has already progressed, so that it can be estimated how much or to what extent polysorbate 20 has already been oxidatively degraded. The degree of oxidative degradation is proportional to the relative or absolute amount or level of the oxidation marker polyethylene glycol-6-laurate in an aqueous formulation containing polysorbate 20. The relative amount or level in a sample means in relation to a reference sample or the amount or level of another sample. The absolute amount or level is the actual amount determined analytically by quantitative analysis.

The term "proportional" in the expression *"the relative peak area [of polyethylene glycol-6-laurate] being proportional to the degree of oxidative degradation of polysorbate 20"* and similar phrases is to be understood as meaning that there is a relationship or correlation between the relative peak area of the oxidation marker polyethylene glycol-6-laurate in a sample and the degree of oxidative degradation of polysorbate 20. In particular, there is a proportional relationship or more precisely an inversely proportional relationship, i.e. the (relative or absolute) amount or level of polyethylene glycol-6-laurate and the (relative or absolute) amount or level of polysorbate 20 are in an anti-correlation with each other, as will be explained in more detail later on.

The expression "level" is to be understood in its broadest sense and means the amount, portion, content, or concentration in which a compound is present in an aqueous liquid, in particular a reference sample or an aqueous formulation. The "relative level" means the amount, portion, content or concentration of a compound expressed in relation or relative to a reference.

The term "aqueous" is intended to mean that water is present in the formulation. For example, water can constitute a part of the solvent, e.g. a small part or the main part of the solvent or can represent the only solvent present. In fact, any type of water may be used. Purified water may be preferred but according to some embodiments also tap water may be used. The type of water selected depends from the intended use of the aqueous formulation. Purified water used according to the present invention is water that has been undergone a purification process such as distillation, reverse osmosis, carbon filtering, capacitive or electro-deionization, micro- or ultrafiltration, ultraviolet oxidation or the like to remove impurities to be suitable for use. Combinations of these processes may also be used in order to achieve water of such high purity, e.g. ultrapure water, that its trace contaminants are measured in parts per billion (ppb) or parts per trillion (ppt). The water used may be also ultrapure water, for example ultrapure water of type 1 (Milli-Q^{®} water) according to ASTM D1193 or ISO 3696. The water used may be also sterile water suitable for administration to a subject such as water for injection (WFI). Also, distilled water, bidistilled or deionized water may be used.

The term "aqueous formulation" refers to a solution in which the solvent or one of the solvents is water. The solution comprises a true solution, dispersion, suspension and the like, unless otherwise stated. The aqueous formulation according to the invention comprises polysorbate and optionally one or more protein(s) and optionally excipient(s).

The term "protein" (used interchangeably with "polypeptide" and "amino acid residue sequence") refers to polymers of any amino acids (naturally occurring or artificial) of any length. The term "protein" includes also proteins that are post-translationally modified through reactions that include, but are not limited to glycosylation, glycation, acetylation, phosphorylation, oxidation, amidation or protein processing.

Modifications and changes, for example fusions to other proteins, amino acid sequence substitutions, deletions or insertions, can be made in the structure of a polypeptide, preferably while the molecule maintains its biological functional activity. For example certain amino acid sequence substitutions can be made in a polypeptide or its underlying nucleic acid coding sequence and a protein with similar properties can be obtained. Amino acid modifications can be prepared for example by performing site-specific mutagenesis or polymerase chain reaction mediated mutagenesis on its underlying nucleic acid sequence. The term "protein" or "polypeptide" thus also includes, for example, fusion proteins consisting of an immunoglobulin component, e.g. the Fc component, and a growth factor, e.g. an interleukin.

The term "protein" includes proteins, polypeptides, fragments thereof, peptides, fusion proteins all of which can be expressed in host cell systems or synthesized via chemical methods. Desired proteins can be for example antibodies, enzymes, cytokines, lymphokines, adhesion molecules, receptors and derivatives or fragments thereof, and any other proteins or polypeptides that can serve as agonists or antagonists and/or have therapeutic or diagnostic use.

Further desired proteins or polypeptides are, for example, but not limited to: insulin, insulin-like growth factor, hGH, tPA, cytokines, such as interleukines (IL), e.g. IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosisfactor (TNF), such as TNF alpha and TNF beta, TNF gamma, TRAIL; G-CSF, GM-CSF, M-CSF, MCP-1, VEGF and single domain antibodies (e.g. derived from camelids). Also included is the production of erythropoietin or any other hormone growth factors and any other polypeptides that can serve as agonists or antagonists and/or have therapeutic or diagnostic use.

Thus, the proteins may be, for example, antibodies, such as a monoclonal antibody, polyclonal antibody, monospecific antibody, multispecific antibody, single chain antibodies or antibody fragments thereof, such as, Fv, scFv, Fab, Fab', scFab, F(ab')2, Fab2, Fc and Fc'-fragments, heavy and light immunoglobulin chains and their constant, variable or hypervariable region as well as Fv- and Fd-fragments, diabody, triabody, scFv-Fc, minibody, or single domain antibody or a mixture thereof.

The term "antibody" as used herein usually refers to a mono-specific antibody, but an antibody may also be multi-specific or fragments thereof. Exemplary antibodies within the scope of the present invention include but are not limited to anti-CD2, anti-CD3, anti-CD20, anti-CD22, anti-CD30, anti-CD33, anti-CD37, anti-CD40, anti-CD44, anti-CD44v6, anti-CD49d, anti-CD52, anti-EGFR1 (HER1), anti-EGFR2 (HER2), anti-GD3, anti-IGF, anti-VEGF, anti-TNFalpha, anti-IL2, anti-IL-5R, anti-IL-36R or anti-IgE antibodies, and are preferably selected from the group consisting of anti-CD20, anti-CD33, anti-CD37, anti-CD40, anti-CD44, anti-CD52, anti-HER2/neu (erbB2), anti-EGFR, anti-IGF, anti-VEGF, anti-TNFalpha, anti-IL2, anti-IL-36R and anti-IgE antibodies.

The term "antibody", "antibodies", or "immunoglobulin(s)" as used herein relates to proteins selected from among the globulins, which are naturally formed as a reaction of the host organism to a foreign substance (=antigen) from differentiated B-lymphocytes (plasma cells). There are various classes of immunoglobulins: IgA, IgD, IgE, IgG, IgM, IgY, IgW. The antibody may be an IgG antibody, such as an IgG1 or an IgG4 antibody. The terms "immunoglobulin" and "antibody" are used interchangeably herein. Antibody include monoclonal, monospecific and multi-specific (such as bispecific or trispecific) antibodies, a single chain antibody, an antigen-binding fragment of an antibody (e.g., an Fab or F(ab')2 fragment), a disulfide-linked Fv, etc. Antibodies can be of any species and include chimeric and humanized antibodies.

The term "sample" is understood as broadly as possible to mean a limited or smaller quantity of the aqueous formulation, which is taken for analysis. The sampling may be done manually or by an automated method.

The term "liquid chromatography-mass spectrometry method" or "LC-MS method" means a method known in the prior art based on a specific analytical chemistry technique combining physical separation by liquid chromatography (or HPLC) with mass analysis by mass spectrometry (MS). This expression is intended to include any technique that combines liquid chromatography and mass spectrometry known by the skilled person. Coupled liquid chromatography-MS systems are known in chemical analysis. These show the advantage that the two methods are usually synergistically improved by combination. An LC-MS system also contains some kind of interface that allows the substances separated by the LC procedure to enter the MS ion source. The interface is necessary because LC instruments and MS instruments are not actually compatible with each other: In an LC system, the mobile phase is under pressure, whereas an MS analyser is usually under high vacuum.

The expression "liquid chromatography-mass spectrometry based method" or "LC-MS based method" is to be understood as a method used according to the present invention comprising liquid chromatography and mass spectrometry, which are carried out directly one after the other, whereby the methods known to the skilled person from the prior art ("LC-MS method" as defined above) serve as a basis. The method of the present invention is based thereon and has been modified and adapted in the manner disclosed herein to meet the desired requirements, particularly to detect and optionally quantify the oxidation marker.

The expressions "quantifying" or "quantification" or the like shall be understood in its broadest sense and stands for the determining of the concentration or amount of the substance polyethylene glycol-6-laurate based on the relative level (relative peak area) found, which is present in an aqueous formulation from which a sample is taken and examined. The quantification is achieved by an external calibration using standard solutions of polyethylene glycol-6-laurate.

The term "recombinant protein" as used herein relates to a protein generated by recombinant techniques, such as molecular cloning. Such methods bring together genetic material from multiple sources or create sequences that do not naturally exist. A recombinant protein is typically based on a sequence from a different cell or organism or a different species from the recipient host cell used for production of the protein, e.g., a CHO cell or a HEK 293 cell, or is based on an artificial sequence, such as a fusion protein. In the context of the present invention the recombinant protein is preferably a therapeutic protein, such as an antibody, an antibody fragment, an antibody derived molecule (e.g., scFv, bi- or multi-specific antibodies) or a fusion protein (e.g., a Fc fusion protein).

The term "expressing a recombinant protein" as used herein refers to a cell comprising a DNA sequence coding for the recombinant protein, which is transcribed and translated into the protein sequence including post-translational modifications, i.e., resulting in the production of the recombinant protein in cell culture.

The term "eukaryotic cell" as used herein refers to cells that have a nucleus within a nuclear envelop and include animal cells, human cells, plant cells and yeast cells. In the present invention an "eukaryotic cell" particularly encompasses mammalian cell, such as Chinese hamster ovary (CHO) cell or HEK293 cell derived cells, and yeast cells.

The term "drug substance" as used herein refers to the formulated active pharmaceutical ingredient (API) with excipients. The API has a therapeutic effect in the body as opposed to the excipients, which assist with the delivery of the API. In the case of biologic therapeutics, the formulated API with excipients typically means the API in the final formulation buffer at a concentration of at least the highest concentration used in the final dosage form, also referred to as drug product.

API(s) may be present in the aqueous formulation. According to the WHO guidelines the "active pharmaceutical ingredient (API)" is any substance or combination of substances used in a finished pharmaceutical product (FPP), intended to furnish pharmacological activity or to otherwise have direct effect in the diagnosis, cure, mitigation, treatment or prevention of disease, or to have direct effect in restoring, correcting or modifying physiological functions in human beings. Furthermore, excipient(s) may be present in the aqueous formulation. The API(s) may be for example one or more proteins. The aqueous formulation containing API(s) is considered as an aqueous biopharmaceutical formulation.

The term "drug product" as used herein refers to the final marketed dosage form of the drug substance for example a tablet or capsule or in the case of biologics typically the solution for injection in the appropriate containment, such as a vial or syringe. The drug product may also be in a lyophilized form.

The term "host cell proteins" (HCPs) refers to process-related protein impurities produced by the host organism during the manufacture and production of biopharmaceuticals. During the purification process, a large proportion, usually >99 % of the impurities, of the HCPs produced are removed from the final product. However, residual HCPs remain in the finished drug product, so that removal of HCPs is one of the biggest challenges for the production of biopharmaceuticals. Therefore, all proteins present in the expression system or substrate beside the protein(s) in question are regarded as impurities and commonly summarized under the term host cell proteins (HCP).

The expressions "comprising", "comprise", "comprised", "containing", "contain" or "contained" shall also encompass the more specific term "consisting of" unless otherwise stated or apparent from the context.

In addition, it should be noted that in this disclosure, the singular and plural forms are not used in a restrictive manner. As used herein, the singular forms "a", "an", "one" and "the" therefore refer to both the singular and the plural, unless otherwise stated or apparent from the context.

The expression "about" or "approximately" means within 10 %, particularly within 5 % and more particularly within 1 % or within 0.1 % of a value specified or an upper or lower range value as indicated. In relation to the mass-to-charge ratio the term "about" has the meaning ± 0.5. That means "about 227 amu" stands for the range of 227 - 0.5 amu to 227 + 0.5 amu or 226.5 to 227.5 amu and "about 487 amu" stands for the range of 487 - 0.5 amu to 487 + 0.5 amu or 486.5 to 487.5 amu.

### EMBODIMENTS OF THE INVENTION

In the following the multi-step methods according to the present invention are described. Optimum method conditions and parameters for each individual step may vary depending on the particular aqueous formulation, optional excipient(s), solvent(s) besides water selected, and optional API(s) present. Unless otherwise specified, the method conditions and parameters of each method step may be readily selected by one of ordinary skill in the art. The experimentation procedures are provided in the experimental section.

Polyethylene glycol-6-laurate was found to be a marker substance giving an indication about the degree of the oxidative degradation of polysorbate 20. Since polyethylene glycol-6-laurate has been identified as a novel marker substance that allows specific monitoring of the oxidative degradation of polysorbate 20, it is also referred to as the oxidative marker or oxidation marker of polysorbate 20 degradation. By using a liquid chromatography-mass spectrometry based method, which has been adapted and modified accordingly for the present invention, the relative level of the marker substance in a sample can be determined and thus the oxidative degradation of polysorbate 20 can be inferred.

The general procedure of the liquid chromatography-mass spectrometry based method according to the present invention is as follows:
At first, a chromatography method is carried out. This is a liquid chromatography process, in particular a HPLC process.

The chromatography procedure is followed by a mass spectrometry method. In the mass spectrometry method, electrospray ionisation (ESI) is normally used to generate ions which are used to determine the molecular mass. In the present method, the polysorbate species are (partially) fragmented in the ESI source, in particular fragments containing the fatty acids are split off. That means 1,3-dioxolanylium ions are produced. In this way, all ions could be measured. Usually, all ions are measured with a mass-to-charge ratio (m/z) in the range of about 100 to about 2000. For evaluation, it is possible that the ion tracks of the detected species are extracted: These are then so-called extracted ion chromatograms or XICs (eXtracted Ion Chromatograms).

Such an extracted ion chromatogram or XIC is generated, for example, for a typical fragment of polysorbate 20 at an ion trace or mass-to-charge ratio of about 227 amu (from m/z 226.5 to m/z 227.5 amu) representing the 1,3-dioxolanylium ion of lauric acid. However, in the present invention, it has been found advantageous if an extracted ion chromatogram XIC is generated at an ion trace or mass-to-charge ratio of a range comprising about 487 amu, particularly comprising or consisting of from m/z 486.5 to m/z 487.5 amu, for data evaluation, which will be explained in detail later on.

The individual steps of the methods according to the invention are now explained in detail below:

### First method, 1. alternative:

In a first alternative of the first method for determining the degree of oxidative degradation of polysorbate 20 in an aqueous formulation containing polysorbate 20, the first sample of the aqueous formulation to be tested is taken as a reference sample.

### - Process step 1

In process step 1 it is provided a first sample of the aqueous formulation to be examined, which serves as a reference sample.

A reference is required because the presence of the oxidation marker polyethylene glycol-6-laurate does not in itself mean that oxidative degradation has occurred, as the aqueous formulation under investigation may already contain the oxidation marker as an impurity. Oxidative degradation can also already take place in the aqueous formulation. If oxidative degradation of polysorbate 20 occurs, it is a continuous process in which an increasing amount of polyethylene glycol-6-laurate is formed. The oxidative degradation can be triggered by a variety of influences, especially environmental influences such as light, the presence of metal ions or the like.

The first sample as reference sample is therefore used to determine a base value and the polyethylene glycol-6-laurate in the sample(s) is determined in relation to the base value.

In this context, it should be noted that the commercial raw material (solid) of polysorbate 20 usually already contains polyethylene glycol-6-laurate, so that this is also present in the aqueous polysorbate 20-containing formulation. If, contrary to expectations, the reference sample does not contain polyethylene glycol-6-laurate, another reference sample is provided.

### - Process step 2

In process step 2 it is provided a second sample and optionally further samples of the aqueous formulation to be examined.

It is therefore possible that more than one sample is taken in step 2, for example two, three, four, five or more further samples of the aqueous formulation to be examined. The number of samples depends on the individual case.

According to an embodiment the samples being taken at successive time intervals T1, T2, T3... one after the other, the times T1, T2, T3... may be of equal or different length. For example, the samples are taken sequentially at time intervals, which may for example be of equal length. For example, in a time interval of 1 month, one sample can be taken, respectively, over a period of several months. In this way, it can be checked over a certain period of time whether oxidative degradation of polysorbate 20 takes place or not and also how much oxidative degradation occurs.

The sampling in step 1 and 2 can be carried out in any procedure known to the expert, for example manually by a person or by an automated device or system. It is a matter of course that the sampling should be carried out in such a way that the following analytical procedure is not adversely affected or compromised. The amount of the sample withdrawn from the aqueous formulation is selected to be large enough to perform a liquid chromatography-mass spectrometry based method with the sample. The skilled person is familiar with the basics of this analytical technique.

### - Process step 3

In the following process step 3, the liquid chromatography-mass spectrometry based method is used to separate polyethylene glycol-6-laurate and to determine the relative peak area of polyethylene glycol-6-laurate, the marker substance for the oxidative degradation of polysorbate 20, in the reference sample of step 1 and each sample of the aqueous formulation of step 2.

The determination of the "relative peak area" or "relative level" of polyethylene glycol-6-laurate, terms used synonymously and interchangeably here, in step 3 means that the polyethylene glycol-6-laurate level is determined in comparison to a reference sample.

In detail, polyethylene glycol-6-laurate is separated from the reference sample (first sample) of step 1, from the second sample and from each optional further sample of step 2 and in each case the peak area of polyethylene glycol-6-laurate in the reference sample, in the second sample and in each optional further sample is determined. Then, the peak area of polyethylene glycol-6-laurate in the second sample and in each optional further sample is determined in relation to the peak area of polyethylene glycol-6-laurate in the reference sample to obtain a relative peak area for the second and each optional further sample.

The relative peak area of polyethylene glycol-6-laurate for the second and each optional further sample in process step 3 may be determined by one of the following two possibilities:
- calculating the difference of the peak areas obtained, wherein the peak area of polyethylene glycol-6-laurate in the reference sample is subtracted, respectively, from the peak area of polyethylene glycol-6-laurate in the second sample and from the peak area of polyethylene glycol-6-laurate in each optional further sample, to obtain the relative peak area(s) for the second sample and each optional further sample(s);
   or
- normalising the peak areas obtained, wherein the peak area of polyethylene glycol-6-laurate in the reference sample is set equal to 100% and the peak area of polyethylene glycol-6-laurate in the second sample and in each of the optional further sample(s) is calculated respectively based thereon.

When the difference is formed, the peak area of the reference sample is subtracted from the peak area of each sample. The peak area of the reference is suitably determined only once and the value obtained (baseline) is then subtracted from the obtained peak area of each sample, respectively. This will be explained by means of an example case:
The following peak areas are determined with the liquid chromatography-mass spectrometry based method:
Peak area for the reference sample: 2000
Peak area for the first sample: 3000.
The relative peak area for the first sample is then: 3000 - 2000 = 1000.
Peak area for a further sample: 4000.
The relative peak area for the further sample is then: 4000 - 2000 = 2000.

No negative values can be obtained because the reference sample is the first sample taken from the aqueous formulation and therefore regularly has an equal or smaller content of polyethylene glycole-6 laurate than the other sample or optional further samples to be taken.

When the peak areas obtained are normalised, the peak area of polyethylene glycol-6-laurate in the reference sample is set equal to 100% and the peak area of polyethylene glycol-6-laurate in the second sample and each optional further sample(s) is calculated respectively based thereon. This will be explained by means of another example case:
The following peak areas are determined with the liquid chromatography-mass spectrometry based method:
Peak area for the reference sample: 2000.
Peak area for the other sample: 3000.
The relative peak area for the reference sample is set to 100%.
The relative peak area for the second sample is then: ((3000x100):2000 =) 150%.
Peak area for a further sample: 4000.
The relative peak area for the reference sample is still set to: 100%.
The relative peak area for the second sample is then: ((4000x100):2000 =) 200%.

Hence, it is only necessary to determine the relative level of polyethylene glycol-6-laurate in a sample of the aqueous formulation compared to a reference in order to determine whether or not oxidative degradation of polysorbate 20 has occurred.

If several samples are taken and analysed, it goes without saying that their polyethylene glycol-6-laurate level is always determined as relative level in comparison with the reference sample (relative peak areas). Therefore, in step 3, the one, two, three, four, five or even more samples are each analysed using a liquid chromatography-mass spectrometry based method: For this purpose, the polyethylene glycol-6-laurate contained in the other sample is separated, its peak area is determined and the relative peak area is determined in relation to the peak area of the reference sample (first sample) using the difference or normalisation as already described to obtain the relative peak area of the other sample. The same procedure is followed for each further sample, i.e. the polyethylene glycol-6-laurate contained in each further sample is separated, respectively, its peak area determined and the relative peak area(s) determined in relation to the reference sample using the difference or normalisation as already described to obtain the relative peak area of each further sample. This procedure is repeated for all samples so that a series of relative peak areas can be determined. According to an embodiment the relative peak areas may be determined as a function of the sampling times T1, T2,....

It is useful if the calculation of the relative peak area in relation to the reference sample is done in the same way in all samples, i.e. if the difference is formed for one sample, it is useful if the difference is used for all samples. If normalisation is used for one sample, then it is appropriate if normalisation is used for all samples. This ensures the comparability of the determination of the relative peak areas of the samples.

The relative peak area for polyethylene glycol-6-laurate is proportional to the degree of oxidative degradation of polysorbate 20. That is, the degree of oxidative degradation is directly related to the determined relative level of the oxidation marker polyethylene glycol-6-laurate in an aqueous formulation to be examined. To the extent that polysorbate 20 is oxidatively degraded, the oxidation marker polyethylene glycol-6-laurate is formed. Based on the determined relative level of oxidation marker, the extent of oxidative degradation of polysorbate 20 can therefore be estimated, in particular how far oxidative degradation has already progressed, so that it is possible to estimate how much or to what extent polysorbate 20 has already been oxidatively degraded. More precisely, there is an inversely proportional relationship (anti-correlation) between the amount or level of polyethylene glycol-6-laurate and the amount or level of polysorbate 20, which will be explained in more detail later.

The samples can therefore be used to determine the course of a possible oxidative degradation. For example, a significantly high level of the oxidation marker present in a sample (e.g. stability sample) relative to the corresponding reference sample represents a high degree of oxidative degradation of polysorbate 20.

In this context, however, it must be taken into account that the proportionality is not given over the entire degradation process of polysorbate 20, since polyethylene glycol-6-laurate itself is degraded over time. Therefore, it is understandable to the person skilled in the art that the teaching of the present invention may relate to the range in which polyethylene glycol-6-laurate has not yet been degraded and the described proportionality is given.

Therefore, the liquid chromatography-mass spectrometry (LC-MS) based method disclosed serves as an analytical tool to determine the level of polyethylene glycol-6-laurate, which corresponds to a certain degree of oxidative degradation of polysorbate 20 in NBE (New Biological Entities) solutions. The method of the invention therefore also allows to distinguish between an oxidative degradation of polysorbate 20 and a non-oxidative degradation, which will also be explained in more detail later-on.

### - Process step 4

In process step 4, following step 3, the polyethylene glycol-6-laurate may be optionally quantified in the sample based on the relative peak area of the second and optionally each further sample. This step is only optional because there may also be cases in which it is sufficient to determine whether oxidative degradation of polysorbate 20 has already taken place or not yet based on the relative level of polyethylene glycol-6-laurate found. However, if a quantitative statement about the extent of the oxidative degradation that has actually taken place is required or desired, a quantitative analysis can be performed in any case.

Quantification takes place in particular via external calibration. For example, the quantification in step 4 may be carried out with an external calibration using standard solutions of polyethylene glycol-6-laurate. This procedure is known to the skilled person, therefore it is not necessary to explain the procedure in all details.

The quantification of polyethylene glycol-6-laurate could be of great use, for example, if the absolute values obtained by the quantification were used for an alignment with established limits and/or ranges.

### - Process step 5

Process step 5 comprises estimating to what extent polysorbate 20 has already been oxidatively degraded in the sample based on an (approximately) inversely proportional relationship between the polyethylene glycol-6-laurate level and the polysorbate 20 level. The degree of oxidative degradation of polysorbate 20 of the sample may be estimated based on the determined relative peak area of polyethylene glycol-6-laurate in step 3 or on the quantity of polyethylene glycol-6-laurate determined in step 4 based on the relative peak area of polyethylene glycol-6-laurate.

It is therefore not only possible to distinguish between non-oxidative degradation and oxidative degradation of polysorbate 20 in aqueous formulation(s), but also the course and progression of oxidative degradation can be determined using the methods of the invention. The (approximately) inversely proportional relationship is shown by the fact that when the (relative) polysorbate 20 level or content in an aqueous formulation in samples is determined over time and when the (relative) polyethylene glycol 6-laurate level or content in an aqueous formulation in samples is determined over time, the determined values are plotted on two curves and compared with each other, then the two curves are (approximately) inversely proportional. Otherwise, the curves are not inversely proportional to each other and no oxidative degradation of polysorbate 20 takes place.

As shown in Figure 2A, which will be described in detail later, there exists a correlation between the decrease in polysorbate 20 concentration due to oxidative degradation and the increase in the relative level of polyethylene glycol-6-laurate formed during oxidative degradation. To an approximation, it can be assumed that the concentration of polysorbate 20 is inversely proportional to the relative level of polyethylene glycol-6-laurate. This indicates that oxidative degradation of polysorbate 20 takes place in Figure 2A. This allows an estimation of the oxidative degradation of polysorbate 20 based on the relative level of polyethylene glycol-6-laurate found in method step 3.

Thus, a high value for the relative level of polyethylene glycol-6-laurate means a high degradation of polysorbate 20 in the investigated sample. A medium value for the relative level of polyethylene glycol-6-laurate means a moderate degradation of polysorbate 20 in the tested sample and a low value for the relative level of polyethylene glycol-6-laurate means a low degradation of polysorbate 20 in the tested sample.

When the estimate for oxidative degradation based on the determined relative polyethylene glycol-6-laurate level is correlated with time, very valuable information about the behaviour of oxidative degradation of polysorbate 20 in an aquous formulation is obtained. This is of particular interest for the stability testing of aqueous formulations, for example during the development, manufacture, shelf life, or storage period thereof, or for aqueous biopharmaceutical formulations, for example to check their quality over time.

It could then also be predicted for an aqueous formulation containing polysorbate 20, particularly an aqueous biopharmaceutical formulation, how the oxidative degradation of polysorbate 20 will most likely develop over time or what level of oxidative degradation of polysorbate 20 can be expected at certain points in time. This could be guided by an estimation of the level of oxidative degradation of polysorbate 20 of an aqueous formulation such as a biopharmaceutical formulation performed using the method of the invention. An identical or very similar biopharmaceutical formulation would behave in the same or correspondingly similar manner. A prediction would therefore be possible and particularly helpful in the development of aqueous formulations, particularly aqueous biopharmaceutical formulations.

### First method, 2. alternative:

In an alternative embodiment of the first method (2. alternative) for determining the degree of oxidative degradation of polysorbate 20 in an aqueous formulation containing polysorbate 20 an external reference sample is used. Except for this difference, all the explanations for the first alternative apply in the same way to the second alternative.

### - Process step 1'

In process step 1' it is provided a reference sample which is an aqueous solution containing polysorbate 20, the polysorbate content being the same as in the aqueous formulation to be examined. For example, a commercially available polysorbate 20 may be used.

Since polysorbate 20 usually already contains polyethylene glycol-6-laurate, an aqueous solution of polysorbate 20 also contains it.

In process step 1' an external reference sample is provided which is used to determine a base value and the polyethylene glycol-6-laurate in the sample(s) is determined in relation to the base value. The explanations for the reference sample in step 1 otherwise apply here mutatis mutandis.

### - Process step 2'

In process step 2' it is provided a first sample and optionally further samples of the aqueous formulation to be examined. It is therefore possible that more than one sample is taken in step 2', for example two, three, four, five or more further samples of the aqueous formulation to be examined. The number of samples depends on the individual case.

According to an embodiment the samples being taken at successive time intervals T1, T2, T3... one after the other, the times T1, T2, T3... may be of equal or different length.

The sampling in step 1' and 2' can be carried out in any procedure known to the expert, for example manually by a person or by an automated device or system. It is a matter of course that the sampling should be carried out in such a way that the following analytical procedure is not adversely affected or compromised. The amount of the sample withdrawn from the aqueous formulation is selected to be large enough to perform a liquid chromatography-mass spectrometry based method with the sample. The skilled person is familiar with the basics of this analytical technique.

### - Process step 3'

In the following process step 3', the liquid chromatography-mass spectrometry based method is used to separate polyethylene glycol-6-laurate and to determine the relative peak area of polyethylene glycol-6-laurate in each sample, whereby instead of the first sample an external reference is used.

In detail, the liquid chromatography-mass spectrometry based method of the invention is used to separate polyethylene glycol-6-laurate from the reference sample of step 1', from the first sample and each optional further sample of step 2' and in each case the peak area of polyethylene glycol-6-laurate in the reference sample, in the first sample and in each optional further sample is determined. Then, the peak area of polyethylene glycol-6-laurate in the first sample and each optional further sample is determined in relation to the peak area of polyethylene glycol-6-laurate in the reference sample to obtain a relative peak area for the first and each optional further sample.

It is convenient not to determine the peak area of polyethylene glycol-6-laurate in the reference sample again for each sample, but to determine the peak area once and use this value (baseline) as a reference. For each sample taken, its peak area is then determined in relation to this value (baseline) for the peak area of the reference.

The relative peak area in process step 3' may be determined by one of the following two possibilities:
- calculating the difference of the peak areas obtained, wherein the peak area of polyethylene glycol-6-laurate in the reference sample is subtracted, respectively, from the peak area of polyethylene glycol-6-laurate in the first sample and from the peak area of polyethylene glycol-6-laurate in each optional further sample(s) to obtain the relative peak area(s) for the first sample and each optional further sample(s);
   or
- normalising the peak areas obtained, wherein the peak area of polyethylene glycol-6-laurate in the reference sample is set equal to 100% and the peak area of polyethylene glycol-6-laurate in the first sample and in each optional further sample(s) is calculated respectively based thereon.

Furthermore, the further explanations of step 3 also apply here.

### - Process steps 4' and 5'

In process step 4' the compound polyethylene glycol-6-laurate may be optionally quantified whereby the relative peak area of the first and each optional further sample is taken as basis. In process step 5' it is possible to estimate to what extent polysorbate 20 has already been oxidatively degraded in the sample based on an inversely proportional relationship between the polyethylene glycol-6-laurate level and the polysorbate 20 level.

The explanations for process steps 4 and 5 apply here in a corresponding manner to process steps 4' and 5'.

### Second method:

In the present Invention it is also provided a (second) method for determining the degree of oxidative degradation of polysorbate 20 in a number of aqueous formulations, wherein in particular each aqueous formulation containing the same amount of polysorbate 20. The number of aqueous formulations is not limited, for example, two, three, four, five or more aqueous formulations may be used, in particular up to 10, up to 20, up to 50 or even more aqueous formulations may be used. The aqueous formulations may differ from each other in terms of their compositions, in particular only the content of polysorbate 20 being chosen to be the same.

### - Process step 1a

In process step 1a it is provided a sample of each aqueous formulation to be examined that respectively contains polysorbate 20. The sampling can be carried out in any procedure known to the expert, for example manually by a person or by an automated device or system. It is a matter of course that the sampling should be carried out in such a way that the following analytical procedure is not adversely affected or compromised. The amount of the sample withdrawn from the aqueous formulation is selected to be large enough to perform a liquid chromatography-mass spectrometry based method with the sample. The skilled person is familiar with the basics of this analytical technique.

It is not necessary to use a reference here, as the peak ares of the samples in this second method can be compared with each other. This is particularly useful when serial tests are carried out. For example, different formulation compositions can be tested for their stability.

According to one embodiment, it is also possible to select a reference sample from the samples taken and to determine the relative peak area of each sample with respect to this reference sample. Which sample is selected as reference sample varies from case to case.

### - Process step 2a

In process step 2a the liquid chromatography and mass spectrometry based method is used for separating polyethylene glycol-6-laurate from each sample of step 1a and determining the peak area of polyethylene glycol-6-laurate in each sample, wherein polyethylene glycol-6-laurate being a marker substance for the oxidative degradation of polysorbate 20. Then, the peak areas of the samples are compared with each other, whereby the change in peak area of the samples being proportional to the degree of oxidative degradation of polysorbate 20.

The relative peak area of polyethylene glycol-6-laurate does not have to be determined here because the samples can be compared directly with each other.

However, in another embodiment, the relative peak area of each sample may be determined relative to a reference sample, the reference sample being one of the samples. Therefore, the explanations for the first and second methods also apply here.

### - Process step 3a

In process step 3a, an optional quantification of the compound polyethylene glycol-6-laurate is carried out on the basis of the obtained peak area of the respective samples. For this purpose an external calibration curve using standard solutions of polyethylene glycol-6-laurate is provided. Such a procedure has already been described in connection with process steps 4 and 4'.

### - Process step 4a

In process step 4a, which can follow step 3a, it can be estimated to what extent polysorbate 20 has already been oxidatively degraded in the sample based on an inversely proportional relationship between the polyethylene glycol-6-laurate level and the polysorbate 20 level, as described in detail in the present description.

Therefore, the second method can also be used to determine the degree of oxidative degradation of polysorbate 20, to distinguish between non-oxidative and oxidative degradation and to determine the course of oxidative degradation over time, although a number of aqueous formulations are examined for this purpose.

The second method can be used for quality control, for example. It is thus possible, for example, to identify particularly stable compositions from a number of different aqueous formulations.

In the following some aspects of the present invention are described in detail.

### Polyethylene qlycol-6-laurate (PEG-6-laurate) as polysorbate 20-specific oxidation marker

In fact, polyethylene glycol-6-laurate is a commonly used and well characterized substance, which has been extensively described in scientific literature and in a variety of patent publications e.g. for its use as detergent, or as educt for diverse esterified compounds [7] and it is also known as additive in cosmetic compositions, e.g. hair conditioner/shampoo compositions [8, 9]. The molecular structure of polyethylene glycol-6-laurate (C₂₄H₄₈O₈) is as follows:

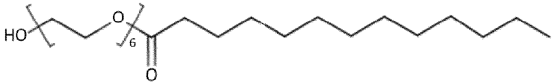

Up to date, however, polyethylene glycol-6-laurate has not been linked to function as analytical oxidative marker substance for polysorbate 20 degradation as described herein. Since polyethylene glycol-6-laurate is a known and well characterised compound, the skilled person is readily able to detect and identify it using the liquid chromatography-mass spectrometry (LC-MS) based method disclosed herein.

The mixture of chemical compounds that are separated here by liquid chromatography are the various degradation products of polysorbate 20, which - as already explained - is in itself already a mixture of different substances. The oxidation marker polyethylene glycol-6-laurate is now to be separated from this mixture and the presence thereof can therefore be detected. To this end the liquid chromatography-mass spectrometry (LC-MS) based method is specially modified and adapted as follows:
The liquid chromatography is especially used as separation chromatography, particularly in the form of column chromatography. In column chromatography, mixtures of chemical compounds are separated using a column with a solid stationary phase which is eluted with one or more mobile phases.

According to the invention, in one embodiment a reversed-phase chromatography is used in liquid chromatography. In reversed-phase chromatography, in contrast to normal-phase chromatography, a stationary phase with non-polar surface properties and a mobile phase with polar properties are used. Known polar solvents for reversed-phase chromatography are e.g. water, methanol, and acetonitrile. Other solvents are also possible.

According to an embodiment of the liquid chromatography-mass spectrometry based method in the liquid chromatography a gradient elution is used. For example, two mobile phases comprising polar solvents are used. For example, water may be used as mobile phase A and acetonitrile, which may advantageously contain formic acid, for example 1 % formic acid, may be used as mobile phase B. The gradient may start with several percent for one of the mobile phases, particularly mobile phase B, for example 1 to 10 %, particularly 1 to 5 %, of mobile phase B, and is ending at 100 % of mobile phase B. The mobile phase A then represents the remaining proportion to 100 %. A linear ramp of the gradient may be used. The skilled person is readily able to find out a suitable composition of the mobile phase or phases and an appropriate gradient which may be used.

It is advantageous if to the sample containing polysorbate 20 to be tested a polar solvent is added prior to perform the liquid chromatography. The polar solvent is particularly the solvent used in liquid chromatography.

In one embodiment of the invention, the liquid chromatography as part of the liquid chromatography-mass spectrometry based method is a high performance liquid chromatography HPLC, also an ultra performance liquid chromatography may be used. The UPLC is more efficient compared to HPLC because it uses a higher pump pressure. The pump pressure for HPLC is usually 40 MPa, for UPLC it is around 100 MPa. UPLC uses stationary phases with smaller particle sizes compared to HPLC, so UPLC allows the separation of smaller particles.

According to one embodiment the liquid chromatography is carried out by high performance liquid chromatography using reversed-phase chromatography, whereby the separation of the different polysorbate species present in polysorbate 20 is carried out on the basis of the increasing hydrophobicity so that the polyethylene glycol-6-laurate may be separated by liquid chromatography using its hydrophobic property. The procedure itself is well known to the expert. The modified and adapted features of the liquid-chromatography-mass spectrometry based method are disclosed herein and the skilled person in the art is readily able to use a known liquid chromatography-mass spectrometry procedure and modify and adapt this procedure according to the present disclosure to separate polyethylene glycol-6-laurate. It is therefore not necessary to explain the procedure in all details. The skilled person in the state of the art is therefore readily able to carry out and optimise the liquid chromatography as part of the liquid chromatography-mass spectrometry (LC-MS) based method on the basis of the disclosed details and explanations given.

After liquid chromatography has been carried out the separated polyethylene glycol-6-laurate can subsequently be identified by means of mass spectrometry coupled with the liquid chromatography.

In an embodiment the mass spectrometry is performed with a heated electrospray ionization (HESI) source.

In a further embodiment the mass spectrometry is selected to be a high resolution-mass spectrometry (HR-MS). For example, the high-resolution mass spectrometry is in particular used for structure elucidation.

In another embodiment, for the data evaluation of the liquid chromatography-mass spectrometry based method of the invention extracted ion chromatograms are generated from a mass-to-charge ratio comprising about 487 amu, particularly a range comprising or consisting of 486.5 to 487.5 amu. Polyethylene glycol-6-laurate surprisingly exhibits a distinct m/z ratio in the mass spectrum that allows highly specific monitoring by liquid chromatography-mass spectrometry-based analysis. This makes it possible to unambiguously identify polyethylene glycol-6-laurate, if present.

The skilled person in the state of the art is easily able to carry out a mass spectrometry method on the basis of the disclosed details and explanations given.

To demonstrate why a mass-to-charge ratio m/z of about 487 amu, particularly a mass-to-charge ratio m/z comprising or consisting of 486.5 to 487.5 amu, is particularly advantageous and especially suitable for detecting polyethylene glycol-6-laurate, reference is made to the spectra as shown in Figures 1A to 1D. Figures 1A to 1D serve to illustrate the autoxidation of polysorbate 20 and exemplify how polyethylene glycol-6-laurate can be determined by separation and detection, in particular can be identified, in a sample using the liquid chromatography-mass spectrometry (LC-MS) based method of the invention:
A sample is prepared consisting of polysorbate 20 in aqueous solution, and an oxidation catalyst in the form of 2,2'-azobis-2-methyl-propanimidamide, dihydrochloride (in the state of the art also referred to as 2,2-azobis(2-methylpropionamidine) dihydrochloride or 2,2'-azobis (2-amidinopropane) dihydrochloride) (AAPH) is added to the sample to induce oxidative degradation in polysorbate 20, i.e. autoxidation of polysorbate 20 is artificially induced. Specifically, oxidative stress is applied to the aqueous polysorbate 20 solution using 1.5 mM 2,2'-azobis-2-methyl-propanimidamide, dihydrochloride (AAPH). The details of the sample preparation are explained in the experimental section under chapter "1.1 Materials", point "c) Sample preparation of artificially stressed polysorbate 20".

The oxidation catalyst AAPH is a water-soluble azo compound known as a model oxidant and free radical generator. As already described polysorbate 20 has a tendency to produce peroxides in situ as the result of an autoxidation. Therefore, AAPH is used to stimulate the autoxidation of polysorbate 20.

Furthermore, another sample is prepared consisting of polysorbate 20 in aqueous solution. No oxidation catalyst is added to the other sample.

The liquid chromatography-mass spectrometry based method of the present invention is used to investigate the samples - one sample with oxidation catalyst present and one sample without oxidation catalyst. The mass spectrometry used as part of the liquid chromatography-mass spectrometry based method is used in full scan mode and the spectra obtained are compared. The details of the liquid chromatography-mass spectrometry based method used are described in the experimental section, under chapter "1.2 Methods", point "c) Quantification of oxidation marker PEG-6 laurate (P6L) via Liquid Chromatography-Mass Spectrometry (LCMS P6L)", but the last step in the described procedure, i.e. the quantification of the polyethylene glycol-6-laurate, is not performed. The results are shown in Figure 1A to 1D.

In Figure 1A, on the left side, extracts of two full-scan total ion chromatograms (TICs) of polysorbate 20 in an aqueous solution resulting from an embodiment of the liquid chromatography-mass spectrometry (LC-MS) based method of the invention are shown at a mass-to-charge ratio m/z of about 227 amu (226.5 to 227.5 amu). A m/z ratio of about 227 amu is used as it corresponds to laurate-esterified compounds which are known to be most prominent in polysorbate 20 i.e. in particular the 1,3-dioxolanylium ion of lauric acid.

In Figure 1A, on the left side, the upper panel shows the sample of the stressed polysorbate 20 solution, i.e. the polysorbate 20 in aqueous solution, to which the oxidation catalyst AAPH is added so that an oxidative degradation of polysorbate 20 is artificially initiated. The lower panel on the left side of Figure 1A shows the unstressed sample of the polysorbate 20 solution, i.e. the polysorbate 20 in aqueous solution, to which no oxidation catalyst AAPH is added.

The table indicated in Figure 1A on the left side, over the upper panel, summarizes the peaks found as well as the peak areas of the spectrum shown on the left side with oxidation catalyst AAPH present (in Figure 1A referred to as "1.5 mM AAPH") and without oxidation catalyst AAPH (in Figure 1A referred to as "No AAPH").

Comparing the unstressed sample (Figure 1A, left side, lower panel) with the AAPH-stressed sample (Figure 1A, left side, upper panel) an increase of a structure, i.e. polyethylene glycol-6-laurate, eluting at a retention time of about 18.4 min by approximately 3.4-fold is observed in the stressed sample.

In Figure 1A, on the right side, there are shown extracts of two full-scan total ion chromatograms (TICs) of polysorbate 20 in an aqueous solution resulting from an embodiment of the liquid chromatography-mass spectrometry (LC-MS) based method of the invention, both shown at a mass-to-charge ratio m/z of about 487 amu (486.5 to 487.5 amu), in the upper chromatogram oxidation catalyst AAPH is added, the lower chromatogram is without oxidation catalyst AAPH. But both chromatograms in Figure 1A, right side, are shown at a mass-to-charge ratio m/z of about 487 amu (486.5 to 487.5 amu). In these chromatograms, it can be seen that the peak at about 18.4 min is surprisingly 20 times higher (Fig. 1A, right side) than the peak at about 18.4 min in the chromatograms at a mass-to-charge ratio m/z of about 227 amu (Fig. 1A, left side). As a result, it can be concluded that the chromatograms have higher selectivity, less interference and higher sensitivity at an m/z ratio comprising or consisting of about 487 amu, as the signal increases about 20-fold at a retention time of about 18.4 min after AAPH stress.

In order to show the differences between the chromatograms based on the data evaluation, reference is made to Figure 1B. In Figure 1B are shown ion chromatograms resulting from an embodiment of the liquid chromatography-mass spectrometry (LC-MS) based method of the invention in oxidatively stressed polysorbate 20 aqueous solution, namely the total ion chromatogram (TIC; upper chromatogram), the extracted ion chromatogram generated at a mass-to-charge ratio m/z of about 227 amu (from m/z 226.5 x to m/z 227.5 amu; middle chromatogram) and the extracted ion chromatogram generated at a mass-to-charge ratio m/z of about 487 amu (from m/z 486.5 x to m/z 487.5 amu; lower chromatogram). In the upper chromatogram several peaks are overlapping. In the middle extracted ion chromatogram generated at a m/z ratio of about 227 amu (226.5 to 227.5 amu) there is interference from probably two peaks at the retention time of about 18.4 min, wherein the peak of the oxidation marker (referred to as "PEG-6-laurate") polyethylene glycol-6-laurate is included. In the lower extracted ion chromatogram generated at a m/z ratio comprising about 487 amu (486.5 to 487.5 amu) there is a single peak of the oxidation marker polyethylene glycol-6-laurate having an excellent selectivity and sensitivity since the mass spectrum is at a m/z ratio comprising about 487 amu. Thus, a m/z ratio comprising or consisting of about 487 amu shows superior selectivity and sensitivity in comparison to a m/z comprising or consisting of about 227 amu. It can therefore be seen that an m/z ratio comprising or consisting of about 227 amu with fragmentation of the free fatty acid in the source is significantly less suitable than an m/z ratio comprising or consisting of about 487 amu without fragmentation in the source. Consequently, a data evaluation of the liquid chromatography-mass spectrometry (LC-MS) based method using a mass-to-charge ratio m/z comprising or consisting of about 487 amu is particularly advantageous and especially suitable for detecting polyethylene glycol-6-laurate in the liquid chromatography-mass spectrometry (LC-MS) based method.

In this regard, it should be noted that the value for the mass-to-charge ratio of 487 amu, rather represents a value of about 487 amu. Therefore, according to the invention, the mass-to-charge ratio is particularly in a range from 486.5 to 487.5 amu which corresponds to about 487 amu.

To demonstrate that the underlying structure of the peak eluting at about 18.4 min, previously identified, does indeed represent polyethylene glycol-6-laurate, the liquid chromatography-mass spectrometry based method using high resolution mass spectrometry is performed. The details of the liquid chromatography-mass spectrometry based method using a high-resolution mass spectrometry are described in the experimental section, under chapter "1.2 Methods", point "b) High resolution-Mass Spectrometry (HR-MS)".

The deconvoluted mass spectrum is shown in Figure 1C, indicating a series of structures having about 443 Da, about 487 Da and about 531 Da. These structures could be identified via accurate determination of monoisotopic peaks as sodium adducts of laurate-esterified POE (polyoxyethylene) chain fragments with 5, 6 and 7 oxy-ethylene units, respectively. The corresponding molecular structures are as follows:
peak at about 443 Da: sodium adduct of C₂₂H₄₄O₇ (PEG-5-laurate),
peak at about 487 Da: sodium adduct of C₂₄H₄₈O₈ (PEG-6-laurate) and
peak at about 531 Da: sodium adduct of C₂₆H₅₂O₉ (PEG-7-laurate).

The polyethylene glycol-6-laurate (herein also referred to as PEG-6-laurate or P6L) shows the highest signal intensity at about 487 Da. It is of particular importance that these three structures identified here correspond to the concepts of the state of the art [1, 2] proposing POE chain reduction as potential oxidation mechanism. Thus, these structures provide the evidence that oxidation of polysorbate 20 occurs via this pathway.

To unambiguously prove the identity and structure of polyethylene glycol-6-laurate by an orthogonal approach (verification of the chemical structure of polyethylene glycol-6-laurate by synthesis and re-analysis) commercially available polyethylene glycol-6-laurate synthesized by ChiroBlock GmbH, Bitterfeld-Wolfen, Germany was purchased and analysed by the liquid chromatography-mass spectroscopy based method of the invention and directly compared with a sample of a stressed polysorbate 20 solution (polysorbate 20 in aqueous solution to which AAPH is added). Figure 1D shows in the upper mass spectrum of commercially available polyethylene glycol-6-laurate (upper panel) and in the lower a mass spectrum a sample of polyethylene glycol-6-laurate from AAPH-stressed polysorbate 20 (lower panel), resulting from an embodiment of the liquid chromatography-mass spectrometry (LC-MS) based method of the invention. As may be derived from Figure 1D the polyethylene glycol-6-laurate in the upper spectrum and lower spectrum at 487.32497 Da and 487.32493 Da, respectively, agree very well with the experimentally determined values, indicating that the commercially available polyethylene glycol-6-laurate corresponds to the experimentally determined oxidation marker polyethylene glycol-6-laurate, confirming the identity of the oxidation marker structure of polyethylene glycol-6-laurate. The different numbers in the last decimal place are due to the measurement inaccuracy but are within measurement variability.

### Correlation between polysorbate 20 degradation and polyethylene glycol-6-laurate concentration

An oxidation marker of polysorbate 20 is increasingly formed with advancing oxidative degradation of polysorbate 20, so that a decreasing polysorbate 20 content can be awaited to be accompanied by an increasing polyethylene glycol-6-laurate content. Actually, it could be confirmed that there is a direct correlation between the oxidative degradation of polysorbate 20 and the detection of a relative level of oxidative marker substance which will be explained in the following.

To investigate the correlation between the relative polyethylene glycol-6-laurate level and an artificially induced degradation of polysorbate 20, polysorbate 20 samples are artificially degraded by adding 2,2'-azobis-2-methyl-propanimidamide, dihydrochloride (AAPH) and a suitable temperature selected to stimulate autoxidation of polysorbate 20. For the preparation of the samples it is referred to the experimental section, under chapter "1.1 Materials", point "c) Sample preparation of artificially stressed polysorbate 20".

In order to evaluate the progress of oxidative degradation in polysorbate 20, samples are taken at different time points. The progress of oxidative degradation in polysorbate 20 is also referred to here as the stress period. In detail, the polysorbate 20 concentration, the relative level of polyethylene glycol-6-laurate and the relative level of intact polysorbate 20 species of artificially oxidized polysorbate 20 are determined at different sampling points. The polysorbate 20 concentration is analysed in the samples via HPLC-CAD and the levels of the oxidation marker polyethylene glycol-6-laurate as well as the intact polysorbate 20 species present in the samples are evaluated via embodiments of the liquid chromatography-mass spectrometry (LC-MS) based method of the invention.

For a detailed description of the analysis of the polysorbate 20 concentration performed by HPLC-CAD it is referred to the experimental section, under chapter "1.2 Methods", point "d) Quantification of polysorbate 20 via High Performance Liquid Chromatography-Charged Aerosol Detection (HPLC-CAD)".

For a detailed description of the analysis of the relative level of the oxidation marker polyethylene glycol-6-laurate via an embodiment of the liquid chromatography-mass spectrometry (LC-MS) based method of the present invention it is referred to the experimental section, under chapter "1.2 Methods", point "c) Quantification of oxidation marker PEG-6 laurate (P6L) via Liquid Chromatography-Mass Spectrometry (LCMS P6L)".

For a detailed description of the analysis of the relative level of the intact polysorbate 20 species present in the samples via an embodiment of the liquid chromatography-mass spectrometry (LC-MS) based method of the present invention it is referred to the experimental section, under chapter "1.2 Methods", point "a) Screening of intact polysorbate 20-species via Liquid Chromatography-Mass Spectrometry (LC-MS POE)".

The results of the analysis are shown in Figure 2A wherein the polysorbate 20 concentration in [mg/mL] (left y axis) and the relative peak area of the oxidative marker (referred to as 'Oxmarker') polyethylene glycol-6-laurate (right y axis) have been plotted against the time in [h]. The polysorbate 20 concentration measured by HPLC-CAD is shown in the curve with triangles, while the relative level of the oxidation marker polyethylene glycol-6-laurate measured by an embodiment of the liquid chromatography-mass spectrometry (LC-MS) based method of the present invention is shown in the curve with circles.

In Figure 2A the polysorbate 20 concentration represented by the curve having triangles shows a continuous decrease from 0.043 mg/mL (0h) to 0.009 mg/mL (35h) which corresponds to a loss of 79 % of polysorbate 20 concentration from the sampling time T₀ (0h) to the sampling time T₆ (35h). This means that throughout the progress of the polysorbate 20 degradation, a continuous decrease in polysorbate 20 concentration was observed. In addition, the HPLC-CAD method has been subjected a validation as stability-indicating HPLC method (data not shown) and it has been found that the degradation of polysorbate 20 using AAPH is reliably and correctly reproduced and demonstrated. As a result, the measured curve (curve having triangles) representing the progress of the polysorbate 20 degradation shows the values reliably and accurately.

Furthermore, the relative level of the oxidation marker polyethylene glycol-6-laurate was evaluated via an embodiment of the liquid chromatography-mass spectrometry (LC-MS) based method of the present invention. The data of the liquid chromatography-mass spectrometry (LC-MS) analysis are also shown in Figure 2A. The reference sample is taken at the time T₀. Consistent and correlated with the detected degradation of polysorbate 20, the relative peak areas of polyethylene glycol-6-laurate represented by the curve having circles shows an increase up to the sampling time point T₄ (12h) (see Figure 2A) and then decreases slightly. After 12h the relative level of polyethylene glycol-6-laurate is decreasing but remaining above the initial level. Then, the relative level of polyethylene glycol-6-laurate again decreases slightly after 24h, which is consistent with the degradation of polysorbate 20 being well advanced by this time. In this connection it should be noted that polyethylene glycol-6-laurate is only an intermediate product of the oxidative degradation of polysorbate 20, which is further degraded, leading to the observed decrease in polyethylene glycol-6-laurate level after 24h. It can therefore indeed be shown that the decrease in the polysorbate 20 concentration correlates with the increase in the relative level of polyethylene glycol-6-laurate.

This means that in a range, which can be readily determined by the skilled person, there is a clear anti-correlation between polyethylene glycol-6-laurate and polysorbate 20, demonstrating the suitability of polyethylene glycol-6-laurate as a direct marker for the artificial oxidative degradation of polysorbate 20.

As already described polysorbate 20 is not a single substance but represents a mixture of a series of different substances and species. The autoxidation of polysorbate 20 affects all existing compounds and species in polysorbate 20. Therefore, it was checked whether the artificially triggered autoxidation and degradation of polysorbate 20 actually takes place and also covers other compounds. Thus, the relative levels of a number of intact polysorbate 20 species which are present in the polysorbate 20 starting material, i.e. polyoxyethylene esters present in polysorbate 20, have been analysed before the degradation of polysorbate 20 (reference, time T₀ = 0h) and during the degradation of polysorbate 20 by an embodiment of the liquid chromatography-mass spectrometry based method of the invention. The results are shown in the block chart of Figure 2B wherein the relative peak areas found of the intact polysorbate 20 species of polysorbate 20 have been plotted against the time in [h]. The intact polysorbate 20 species analysed are POE-sorbitan monoC12, POE-isosorbide monoC12, POE monoC12 and POE-sorbitan diC12, the chemical structures thereof are shown in Figure 3 (cf. [10]).

Figure 2B shows a continuous decrease in relative levels (relative peak areas) for the species POE-sorbitan monoC12, POE-isosorbide monoC12 and POE-sorbitan diC12, all of which are decreasing with increasing duration of oxidative stress. The greatest decrease is observed in the species POE-sorbitan diC12 which was found to be susceptible to oxidative degradation in polysorbate 20 during experiments (data not shown). In other words the degradation of the polysorbate 20 in solution is confirmed by the relative levels of the intact polysorbate 20 species found by an embodiment of liquid chromatography-mass spectrometry (LC-MS) based method as shown in Figure 2B.

Therefore, it exists a clear anti-correlation between polyethylene glycol-6-laurate and intact polysorbate 20 levels, demonstrating the suitability of polyethylene glycol-6-laurate as a direct marker for the artificial oxidative degradation of polysorbate 20.

### The aqueous formulation comprising excipient(s) and optionally other ingredient(s)

It has been found that polyethylene glycol-6-laurate serves as authenthic oxidation marker for artificially oxidative stressed samples. In order to be used as an oxidation marker, it should be possible to determine it reliably and correctly without the artificial influence of a supplemented catalyst under ambient conditions, especially also in aqueous biopharmaceutical formulations, so that the different degradation pathways of polysorbate 20 can be distinguished. In this connection it is also important to assure that formulation excipients typically present, for example in aqueous biopharmaceutical formulations, do not interfere with the determination of polyethylene glycol-6-laurate.

In fact, it has been found that the presence of polyethylene glycol-6-laurate as an oxidation marker in the oxidative degradation of polysorbate 20 does not only apply to an artificially produced oxidative degradation, but this oxidation marker also occurs during the naturally occurring oxidative degradation of polysorbate 20 in aqueous formulations. It was also found that the presence of various usual formulation excipients in the aqueous formulation does not interfere with the determination of polyethylene glycol-6-laurate and does not adversely affect the method of the invention in any way.

Furthermore, also the presence of other igredient(s) such as one or more proteins does not impair the determination of polyethylene glycol-6-laurate.

The proteins that can advantageously be used are for example selected from a monoclonal antibody, polyclonal antibody, monospecific antibody, multispecific antibody, single chain antibodies or antibody fragments thereof, such as, Fv, scFv, Fab, Fab', scFab, F(ab')2, Fab2, Fc and Fc'-fragments, heavy and light immunoglobulin chains and their constant, variable or hypervariable region as well as Fv- and Fd-fragments, diabody, triabody, scFv-Fc, minibody, or single domain antibody or a mixture thereof.

The aqueous formulation may therefore be an aqueous formulation comprising usual formulation excipients. This can be an aqueous formulation that can be used in the biopharmaceutical field, which comprises one or more excipient(s) and optionally one or more other ingredient(s) such as protein(s).

### The aqueous formulation as biopharmaceutical formulation

According to an embodiment the aqueous formulation may be an aqueous biopharmaceutical formulation. The aqueous biopharmaceutical formulation may contain pharmaceutically acceptable polysorbate 20, one or more pharmaceutically acceptable excipient(s), and optionally one or more active pharmaceutical ingredient(s) (APIs). Examples of pharmaceutically acceptable polysorbate 20 has been already described. Pharmaceutically acceptable excipient(s) which may be used in biopharmaceutical formulations are well-known by the skilled person.

The one or more active pharmaceutical ingredient(s) (APIs) may be, for example, one or more proteins selected from therapeutic protein(s), which are used for the prevention or treatment of a disease or a disorder.

The oxidation marker can therefore also be used relating to aqueous biopharmaceutical formulations to determine whether or not oxidative degradation of polysorbate 20 has already occurred. That the method of the invention also functions in aqueous biopharmaceutical formulations is demonstrated and confirmed by the following investigations.

The oxidative degradation of polysorbate 20 has been investigated in a number of aqueous biopharmaceutical formulations. For this purpose two different monoclonal antibody formulations, referred to as mAb-1 formulation and mAb-2 formulation, and a placebo to mAb-1 formulation were used. The placebo to mAb-1 formulation is the same aqueous biopharmaceutical formulation as the mAb-1 formulation but without the presence of the monoclonal antibody mAb-1.

Both mAb formulations as well as the placebo formulation are aqueous formulations and contain pharmaceutically acceptable polysorbate as well as pharmaceutically acceptable excipients. Detailed information on the mAb-1 and mAb-2 monoclonal antibodies and the composition of the aqueous biopharmaceutical formulations, namely mAb-1 formulation and mAb-2 formulation as well as the placebo to mAb-1 formulation, can be found in the experimental section under chapter "1.1 Materials", point "a) Monoclonal antibodies".

In addition, mAb-1 used in the mAb-1 formulation was prepared by different process variants, namely process variant A and process variant B, whereby the downstream process of process variant B was optimized regarding the removal of host cell impurities. Therefore, the mAb-1 formulation was examined in two alternatives, with process variants A and B.

Also mAb-2 used in the mAb-2 formulation was prepared by different process variants, namely process variant A and process variant B. Process variant A was performed on a laboratory scale, process variant B was performed on a biopharmaceutical plant. Therefore, the mAb-2 formulation was also examined in two alternatives, with process variants A and B.

The monoclonal antibodies mAb-1 and mAb-2 are both therapeutic proteins, which are used for the prevention or treatment of a disease or a disorder.

In order to investigate the oxidative degradation of polysorbate 20 over time, the mAb-1 formulation described above was stored at room temperature (25 °C) for 3 months without addition of any artificial oxidation catalyst such as AAPH; the mAb-2 formulation was stored at room temperature (25 °C) for 6 months without addition of any artificial oxidation catalyst such as AAPH.

At first, the polysorbate 20 concentration and relative levels of polyethylene glycol-6-laurate oxidation marker for the mAB-1 formulation and a corresponding placebo formulation were determined at different sampling times. The results are shown in Figures 4A and 4B.

Figure 4A shows the polysorbate 20 concentration in [mg/mL] plotted against the time in [months] as determined by HPLC-CAD analysis. Details of the HPLC-CAD analysis can be found in the experimental section, under chapter "1.2 Methods", point "d) Quantification of polysorbate 20 via High Performance Liquid Chromatography-Charged Aerosol Detection (HPLC-CAD)".

Figure 4B shows the relative levels of polyethylene glycol-6-laurate oxidation marker based on the relative peak areas in [%] plotted against the time in [months] as determined in the liquid chromatography-mass spectrometry (LC-MS) based method of the invention. For details of the method it is referred to the experimental section, under chapter "1.2 Methods", point "c) Quantification of oxidation marker PEG-6 laurate (P6L) via Liquid Chromatography-Mass Spectrometry (LCMS P6L)".

In Figure 4A, both mAb1 formulations, wherein mAb-1 has been produced using the two different process variants A and B, show a decrease in polysorbate 20 levels, respectively, albeit at different kinetics. Also the pacebo formulation shows a decrease of the polysorbate 20 levels (curve having triangles).

In detail, in Figure 4A, the mAb-1 formulation, which originates from process variant A (the curve having circles: mAb-1 (process variant A)), shows a significant loss of total polysorbate 20 during storage, measured by HPLC-CAD. In contrast to process variant A, in process variant B (the curve having squares: mAb-1 (process variant B)) the downstream process was optimized regarding the removal of host cell impurities and thus yields a mAb-1 formulation that is virtually stable during storage and does show a polysorbate 20 degradation which is minimized as much as possible.

In Figure 4B only the placebo formulation (the curve having triangles: placebo to mAb-1 formulation) shows an increase in the relative levels of the polyethylene glycol-6-laurate oxidation marker. Furthermore, as may be seen in Figure 4B both process variants A and B of the mAb-1 formulations equally show no increase in the relative levels of polyethylene glycol-6-laurate. However, when compared with the corresponding placebo formulation, a significant loss of polysorbate 20 in the placebo formulation is observed (in Figure 4A: the curve with the triangles) combined with a concurrent increase in the relative levels of polyethylene glycol-6-laurate (in Figure 4B: also the curve with the triangles). Since degradation triggered by host cell proteins (HCP) can be excluded in the placebo formulation, the loss of polysorbate 20 must be due to oxidative degradation.

Furthermore, since both process variants A and B of the mAb-1 formulations do not show an increase in the relative levels of polyethylene glycol-6-laurate as shown in Figure 4B, an oxidative degradation of polysorbate 20 does not take place in both mAb-1 formulations. Furthermore, it can be assumed from Fig. 4A and 4B that the polysorbate 20 in the mAB-1 formulation, process variant A, is not subject to oxidative but to hydrolytic degradation.

As a result, Figures 4A and 4B clearly demonstrate that it is possible to distinguish between non-oxidative degradation and oxidative degradation in the degradation of polysorbate 20 based on the relative levels of polyethylene glycol-6-laurate found.

In fact, stability studies confirm the above conclusions. For this reasons stability studies were performed. The placebo to mAb-1 formulation and the mAb-1 formulation prepared according to process variant A were analysed using liquid chromatography-mass spectrometry (LC-MS) methods. The data found are indicated in the bar charts of Figures 5A and 5B. Figures 5A and 5B show the stability studies of the formulations during 3 months, wherein the relative peak areas plotted against the time in [months] were analysed by LC-MS-POE according to an embodiment of the liquid chromatography-mass spectrometry (LC-MS) based method of the invention and by LC-MS-FFA according to a liquid chromatography-mass spectrometry (LC-MS) method. The details for the LC-MS-POE and LC-MS-FFA methods are explained in the experimental section (chapter "1.2 Methods", point "a) Screening of intact polysorbate 20-species via Liquid Chromatography-Mass Spectrometry (LC-MS POE)" and chapter "1.2 Methods", point "e) Determination of Free Fatty Acids (FFA) via Liquid Chromatography-Mass Spectrometry (LC-MS FFA)"). The datas for the LC-MS-FFA are not indicated.

Figure 5A shows a block chart of the content levels of the intact polysorbate 20 species as relative peak areas in [%] during 3 months representing a stability study of the placebo to mAb-1 formulation. Again, the intact polysorbate 20 species are the compounds present in polysorbate 20 before the oxidative polysorbate 20 degradation occurs (reference, start) and during the oxidative degradation of polysorbate 20 observed up to 3 months. The intact polysorbate 20 species analysed are again POE-sorbitan monoC12, POE-isosorbide monoC12, POE monoC12 and POE-sorbitan diC12, the chemical structures thereof are shown in Figure 3 (cf. [10]).

In Figures 5A and 5B, the first sample of the aqueous formulation is not taken as a reference sample, but a separate reference sample is used. The separate reference sample is polysorbate 20 (commercial available raw material) in aqueous solution (not shown in the figure). For determination of the relative peak areas the normalisation procedure as described is used. Since a separate reference sample is used, the relative peak areas of the start sample are not 100%.

Figure 5B shows a block chart of the relative levels of the intact polysorbate 20 species as relative peak areas in [%] during 3 months representing a stability study of the mAb-1 formulation (process variant A). A comparison of the profiles of intact polysorbate 20 species over the stability storage period show a decrease of all species for the mAb-1 formulation (process variant A) in Figure 5B, while the corresponding placebo formulation only shows a clear decrease of POE-sorbitan diC12.

The greater decrease of POE-sorbitan diC12 in polysorbate 20 of the placebo formulation (Figure 5A) compared to the mAb-1 formulation (Figure 5B) can be explained by the fact that the di-esterified polysorbate 20 species such as POE-sorbitan diC12 are less susceptible to enzymatic degradation due to steric hindrance, whereas they typically decrease at high rates during oxidative degradation (see Figure 5A) [1].

Due to the increase of the relative level of polyethylene glycol-6-laurate in the placebo formulation as shown in Figure 4B this clearly indicates an oxidative degradation of polysorbate 20 in the placebo formulation.

Thus, polyethylene glycol-6-laurate can also serve as marker substance for the presence of oxidative degradation pathways in biopharmaceutical formulations.

Therefore, the relative levels of polyethylene glycol-6-laurate can be detected in these aqueous biopharmaceutical formulations under appropriate storage conditions in the absence of artificial oxidation catalysts, showing that polyethylene glycol-6-laurate is also a reliable oxidation marker in native biopharmaceutical formulations.

Numerous other experiments were conducted, all confirming that polyethylene glycol-6-laurate is a reliable oxidation marker for the oxidative degradation of polysorbate 20, which can also be determined in aqueous biopharmaceutical formulations independent of the presence of usual excipients and/or active pharmaceutical ingredients (APIs).

In order to demonstrate that polyethylene glycol-6-laurate is effectively a reliable oxidation marker in native aqueous biopharmaceutical formulations, the results of another monoclonal antibody containing aqueous biopharmaceutical formulation, namely the mAb-2 formulation, are shown in the following (detailed information to the monoclonal antibody mAb-2 as well as the composition of the aqueous biopharmaceutical formulations, namely mAb-2 formulation are indicated in the experimental section under chapter 1.1, point a)).

In this case, mAb-2 formulations were investigated, wherein mAb-2 has been prepared with two different processes, namely variant A and variant B. Process variant A was carried out on a laboratory scale and process variant B was carried out in a biopharmaceutical plant. The data are shown in Figures 6A and 6B.

Figure 6A shows the polysorbate 20 concentration in [mg/mL] plotted against the time in [months] as determined by HPLC-CAD analysis (for details it is referred to the experimental section, under chapter 1.2, point d)). Figure 6B shows the relative level of the polyethylene glycol-6-laurate oxidation marker based on the relative peak areas in [%] plotted against the time in [months] as determined in the liquid chromatography-mass spectrometry (LC-MS) based method (for details it is referred to the experimental section, under chapter 1.2, point c)).

As may be seen in Figure 6A the mAb-2 formulation prepared by process variant A and the mAb-2 formulation prepared by process variant B show comparable rates of total polysorbate 20 degradation, respectively, albeit at different kinetics. However, as shown in Figure 6B, an increase in the relative levels of polyethylene glycol-6-laurate was only detected for process variant A of the mAb-2 formulation (the curve having triangles), indicating that both process variants differ in their degradation pathways, whereby only process variant A shows an increase of the relative level of polyethylene glycol-6-laurate during stability storage indicating an oxidative degradation of polysorbate 20.

Therefore, also Figures 6A and 6B demonstrate that it is possible to distinguish between non-oxidative degradation and oxidative degradation in the degradation of polysorbate 20 in aqueous biopharmaceutical formulations, based on the relative level of polyethylene glycol-6-laurate found. The determination of the relative level of polyethylene glycol-6-laurate by separation and detection thereof is possible independent of the presence of one or more excipients and independent of the presence of one or more proteins, for example therapeutic proteins.

Again, this corresponds well with LC-MS characterization data for stability studies of the process variants A and B of the mAb-2 formulation found by LC-MS-POE and LC-MS-FFA (details for the LC-MS-POE and LC-MS-FFA methods are explained in the experimental section, under chapter 1.2, point a) and under chapter 1.2, point e)). The data for LC-MS-FFA are not shown.

The results are shown in Figure 7A (process variant B) and Figure 7B (process variant A). In Figures 7A and 7B, unlike Figures 5A and 5B, the first sample of the aqueous formulation under investigation is used as the reference sample. For determination of the relative peak areas the normalisation procedure as described is used. Since the first sample serves as reference sample, the relative peak areas of the starting sample = 100%.

In Figures 7A and 7B both process variants of the mAb-2 formulation show a decrease of intact polysorbate 20 species. However, only process variant A (Figure 7B) shows an increase of the relative levels of polyethylene glycol-6-laurate during stability storage indicating an oxidative degradation of polysorbate 20 as shown in Figure 6B (curve having triangles). The oxidative degradation is confirmed by a decrease in the di-esterified species POE-sorbitan diC12 for process variant A of the mAb-2 formulation (Figure 7B) because the di-esterified polysorbate 20 species are less susceptible to enzymatic degradation due to steric hindrance, but typically show a decrease during oxidative degradation [1].

As further evidence that polyethylene glycol-6-laurate is indeed a reliable oxidation marker in native aqueous biopharmaceutical formulations, in Figures 8A and 8B the results of another monoclonal antibody containing aqueous biopharmaceutical formulation, namely the mAb-4 formulation and placebo to mAb-4 formulation, are shown (detailed information to the monoclonal antibody mAb-4 as well as the composition of the aqueous biopharmaceutical and placebo formulations and the stability conditions are indicated in the experimental section under chapter 1.1, points a) and b)).

Figure 8A shows the polysorbate 20 concentration in [mg/mL] plotted against the time in [months] as determined by HPLC-CAD analysis (for details it is referred to the experimental section, under chapter 1.2, point d)). Figure 8B shows the relative level of the polyethylene glycol-6-laurate oxidation marker based on the relative peak areas in [%] plotted against the time in [months] as determined in the liquid chromatography-mass spectrometry (LC-MS) based method (for details it is referred to the experimental section, under chapter 1.2, point c)).

As may be seen in Figure 8A, the mAb-4 formulation (curve with triangles) shows virtually no decrease in concentration of intact polysorbate 20 species, while the placebo to mAb-4 formulation containing no antibody (curve with dots) shows a significant decrease in concentration of intact polysorbate 20 species during stability storage. The two curves therefore show that only in the placebo formulation there is a clear degradation of polysorbate 20, with polysorbate 20 being practically completely degraded after 6 months.

Figure 8B shows according to the LC-MS based method of the invention that polyethylene glycol-6-laurate is formed as a degradation product of polysorbate 20 to a high extent for the placebo formulation, whereas the mAb formulation shows almost no increase in PEG-6-laurate levels. The curves of figures 8A and 8B therefore anti-correlate with each other, also temporally.

Therefore, polyethylene glycol-6-laurate serves as a true oxidation marker not only under artificial conditions but also in native aqueous formulations such as biopharmaceutical formulations and stability tests of samples. The marker substance, particularly the relative level thereof, is capable of indicating oxidative degradation both in presence and absence of one or more active pharmaceutical ingredients (APIs) such as proteins, particularly monoclonal antibodies.

The oxidation marker polyethylene glycol-6-laurate makes it also possible to distinguish between non-oxidative degradation and oxidative degradation in the degradation of polysorbate 20 in aqueous formulations, particularly aqueous biopharmaceutical formulations, with or without one or more active pharmaceutical ingredients (APIs) and with or without one or more excipients.

### Method performance

The liquid chromatography-mass spectrometry (LC-MS) based method of the invention was therefore adapted to be used for the separation, detection, identification of the polyethylene glycol-6-laurate, particularly in view of the relative peak area or relative level thereof and optionally quantification of polyethylene glycol-6-laurate. In order to verify that the liquid chromatography-mass spectrometry (LC-MS) based method is a viable and reliable test method, the method was investigated in terms of linearity, specificity, trueness, robustness and precision according to known prior art data [11, 12].

It was found to be a suitable method providing linear, precise, accurate and specific reliable results with respect to the relative level of polyethylene glycol-6-laurate present. The details are given in the experimental section (under chapter "2. Characterization of method performance of LC-MS P6L" and in Figure 8).

### Further subjects of the present invention

According to the present invention it is also provided a process (third method) of producing a recombinant protein, said process comprising the following steps:

| | |
|---|---|
| step a) | cultivating in a cell culture an eukaryotic cell expressing a recombinant protein; |
| step b) | harvesting the recombinant protein; |
| step c) | purifying the recombinant protein using an aqueous formulation; |
| step d) | using polysorbate 20, formulating the recombinant protein into a pharmaceutically acceptable aqueous formulation suitable for administration; and |
| step e) | obtaining at least one sample of the pharmaceutically acceptable aqueous formulation of step d); |

wherein the process (third method) additionally comprises performing a method (first or second method) for determinig the degree of oxidative degradation of polysorbate 20 as disclosed in the present invention with the sample obtained in step e).

According to an embodiment the above process (third method) for producing a recombinant protein comprises obtaining at least one sample containing the recombinant protein and polysorbate 20 in step e) as described above, wherein the sample from step d) is a drug substance sample or a drug product sample.

The recombinant protein of the above process of the invention is produced in eukaryotic cells, following expression, the recombinant protein is harvested and further purified. The recombinant protein may be recovered from the culture medium as a secreted protein in the harvested cell culture fluid (HCCF) or from a cell lysate (i.e., the fluid containing the content of a cell lysed by any means, including without being limited thereto enzymatic, chemical, osmotic, mechanical and/or physical disruption of the cell membrane and optionally cell wall) and purified using techniques well known in the art. The recombinant protein is then formulated into a pharmaceutically acceptable aqueous formulation suitable for administration using polysorbate 20 and at least one sample containing the recombinant protein and polysorbate 20 of the formulated recombinant protein is taken.

The above described process (third method of the invention) additionally comprises performing the method (first or second method of the invention) for determining the degree of oxidative degradation of polysorbate 20 as disclosed in the present invention with the sample obtained in step e).

The present invention is also directed to the use of polyethylene glycol-6-laurate as oxidation marker in a method in connection with the oxidative degradation of polysorbate 20 in an aqueous formulation or a number of aqueous formulations that respectively contain polysorbate 20, whereby the method is the (first or second) method of the present invention as disclosed herein.

According to the present invention the (first or second) method as disclosed herein may be used as a stability testing during the development, manufacture, shelf life, or storage period of an aqueous formulation or a number of aqueous formulations that respectively contain polysorbate 20 for determining the oxidative degradation of polysorbate 20 in the aqueous formulation(s).

The advantages of the present invention are manifold:
Polysorbate 20 is of particular importance due to its known function to stabilising ingredients present in aqueous formulations whereby its known autoxidation leading to oxidative degradation and consequent impairment of the aqueous formulations. Monitoring the oxidative degradation of polysorbate 20 is now possible for the first time using a compound that indicates degradation. This is polyethylene glycol-6-laurate, which is an oxidation marker for the oxidative degradation of polysorbate 20. In the state of the art, oxidation markers for polysorbate 20 have been postulated based on various proposed degradation mechanisms, but these have not yet been experimentally identified as true components of the oxidative degradation process of polysorbate 20.

It can be demonstrated that polyethylene glycol-6-laurate is indeed an oxidation marker for polysorbate 20-containing solutions when autoxidation of polysorbate 20 is artificially produced by the addition of an oxidation catalyst. However, polyethylene glycol-6-laurate specific for oxidative degradation also occurs during storage of aqueous formulations containing polysorbate 20 without the addition of oxidation catalyst, i.e. when a natural oxidative degradation of polysorbate 20 takes place.

Therefore, polyethylene glycol-6-laurate not only serves as authenthic oxidation marker for artificially stressed samples but it is also a reliable and correct oxidation marker in aqueous formulations, for example aqueous biopharmaceutical formulations.

According to the invention, a liquid chromatography-mass spectrometry (LC-MS) based method is provided that enables reliable determination by separation and detection of the relative level of the oxidation marker polyethylene glycol-6-laurate in an aqueous formulation. The liquid chromatography-mass spectrometry (LC-MS) based method is based on an existing method, which has been modified and adapted for the oxidation marker polyethylene glycol-6-laurate according to the invention.

The liquid chromatography-mass spectrometry (LC-MS) based method for the determination of the relative levels of polyethylene glycol-6-laurate functions in simple aqueous solutions containing only polysorbate 20 as well as in very complex aqueous formulations containing excipient(s) as well as biopharmaceutical formulations, with and without active pharmaceutical ingredients (APIs). The biopharmaceutical formulations can also include typical excipients such as buffers, sugars, stabilisers and the like, which do not interfere with the determination of the relative level of the oxidation marker.

Independent of the presence of the oxidation marker, the occurrence of oxidative degradation in polysorbate 20 has been confirmed by stability studies independent of the methods of the invention. In such stability studies, a decrease in all intact polysorbate 20 species is observed, with a significant decrease in one di-ester species. These di-ester species are known to be less susceptible to enzymatic degradation due to steric hindrance, but typically decrease at high rates upon oxidative degradation, thus confirming oxidative degradation.

The liquid chromatography-mass spectrometry (LC-MS) based analytical method therefore enables monitoring of the relative levels of polyethylene glycol-6-laurate even in complex aqueous formulations, where monitoring of the relative levels of polyethylene glycol-6-laurate as an oxidation marker allows distinguishing between hydrolytic and oxidative degradation of polysorbate 20 in the aqueous formulations.

In fact, the developed liquid chromatography-mass spectrometry (LC-MS) based method of the invention allows that relative levels of polyethylene glycol-6-laurate can be measured linearly, accurately and precisely even in complex aqueous formulations.

The increase in the relative levels of polyethylene glycol-6-laurate marker substance correlates well with the loss of polysorbate 20 during oxidative degradation, both when oxidation is artificially induced and when oxidation of polysorbate 20 occurs naturally. Therefore, according to the present invention it is possible to distinguish between non-oxidative degradation and oxidative degradation in the degradation of polysorbate 20 in aqueous formulations. In case a degradation of polysorbate 20 is observed which correlates with a simultaneous increase in the relative levels of polyethylene glycol-6-laurate, there is clearly an oxidative degradation of the polysorbate 20. In case a degradation of polysorbate 20 is observed but a simultaneous increase in the relative levels of polyethylene glycol-6-laurate does not occur, then there is clearly a non-oxidative degradation of polysorbate 20. So far, no analytical tools have been described in the prior art that would allow a distinction between hydrolytic and oxidative degradation of polysorbate 20 by direct analytical means.

Thus, the relative levels of polyethylene glycol-6-laurate serve as marker for the presence of oxidative degradation pathways in aqueous formulations. The oxidative marker is also capable of indicating oxidative degradation in aqueous biopharmaceutical formulations both in presence and absence of active pharmaceutical ingredients (API) such as proteins, for example monoclonal antibodies.

An additional quantification of the oxidation marker polyethylene glycol-6-laurate also makes it possible to estimate the degree of oxidative degradation and thus draw conclusions about the polysorbate 20 still present in an aqueous formulation.

It is even possible, based on the methods of the invention, to make predictions about the expected oxidative degradation of the polysorbate 20 of an identical or very similar aqueous formulation based on an estimate of the oxidative degradation of the polysorbate 20 of an aqueous formulation at a particular time. This can be particularly important in the development of aqueous formulations, particularly aqueous biopharmaceutical formulations.

In the present Invention it is also possible to determine the degree of oxidative degradation of polysorbate 20 in a number of aqueous formulations, wherein in particular each aqueous formulation containing the same amount of polysorbate 20. This allows serial tests to be carried out on a large number of aqueous formulations, for example, enabling quality control or stability control. In this way, optimised formulation compositions can also be created.

The invention therefore provides for the first time methods that use an analytical method to monitor, detect and optionally quantify a previously undescribed analyte, namely polyethylene glycol-6-laurate, in aqueous formulations, for example aqueous biopharmaceutical formulations, to observe and estimate the oxidative degradation of polysorbate 20.

### List of abbreviations:

| | |
|---|---|
| AA | Automatic Area (indicates that an automatic integration of the peak has been made) |
| AAPH | 2,2'-azobis-2-methyl-propanimidamide, dihydrochloride |
| ACN | acetonitrile |
| amu | atomic mass unit |
| API | active pharmaceutical ingredient |
| CAD | charged aerosol detector |
| EIC or XIC | extracted ion chromatogram |
| ESI | electrospray ionization |
| FFA | free fatty acid |
| HCP | host cell protein |
| HESI | heated electrospray ionization |
| HP | high purity |
| (HP)LC | (high performance) liquid chromatography |
| LOD | limit of detection |
| LOQ | limit of quantification |
| MA | Manual Area (indicates that the integration of the peak was done manually by the user) |
| mAb | monoclonal antibody |
| MP | mobile phase |
| MS | mass spectrometry |
| NBE | new biological entities |
| n.c. | not calculated |
| n.d. | not detected |
| Oxmarker | oxidative marker or oxidation marker |
| P6L or PEG-6-laurat | polyethylene glycol-6-laurate |
| PEG | polyethylene glycol |
| POE | polyoxyethylene |
| PS | polysorbate |
| PS20 | polysorbate 20 |
| PS20 HP | polysorbate 20 high purity (commercial product) |
| RH | relative humidity |
| RP | reversed-phase |
| RT | retention time |
| TIC | total ion count or total ion chromatogram |

Details of the experiments carried out are given below. The following detailed information on experiments is not to be understood as a limitation of the present invention.

### EXPERIMENTATION

### 1. MATERIALS AND METHODS

### 1.1 Materials

All experiments were conducted with polysorbate 20 high purity (PS20 HP) (Croda, Edison, NJ, USA). As polyethylene glycol-6-laurate (PEG-6 laurate), formula C₂₄H₄₈O₈, molecular weight 464.65 g/mol, synthetic polyethylene glycol-6-laurate of a purity of 95 % was used synthesized by ChiroBlock GmbH, Bitterfeld-Wolfen, Germany). All other chemicals used were of analytical grade and obtained from commercial sources.

### a) Monoclonal antibodies

Four monoclonal antibodies (mAbs) in specific formulations were used:
The mAb-1 formulation contains mAb-1, which is a type IgG1 antibody.

The mAb-1 used in the mAb-1 formulation was prepared by different process variants, namely process variant A and process variant B, whereby the downstream process of process variant B was optimized regarding the removal of host cell impurities.

The process variant A of mAb-1 formulation is concentrated at 65 mg/mL and formulated with 0.200 g/mL polysorbate 20, 11.4 mM L-histidine, 12.8 mM L-histidine HCl + 2H₂O, 213.0 mM mannitol, 28.3 mM sucrose, pH 6.0.

The process variant B of mAb-1 formulation is concentrated at 65 mg/mL and formulated with 0.200 g/mL polysorbate 20, 11.4 mM L-histidine, 12.8 mM L-histidine HCl + 2H₂O, 43.9 mM mannitol, 184 mM sucrose, pH 6.0.

The mAb-2 formulation consisting of mAb-2, which is a type IgG1 antibody, concentrated at 50 mg/mL and formulated with 0.400 mg/mL polysorbate 20, 4.23 mM acetic acid, 15.8 mM sodium acetate trihydrate, 220 mM glycine, 20 mM trehalose dihydrate, pH 5.5.

The mAb-3 formulation consisting of mAb-3, which is a type IgG1 antibody, concentrated at 60 mg/mL and formulated with 0.400 mg/mL polysorbate 20, 45 mM acetate, 150 mM sucrose, 25 mM L-arginin HCL, pH 5.5.

The placebo to mAb-1 formulation is a formulation containing 0.200 g/mL polysorbate 20, 11.4 mM L-histidine, 12.8 mM L-histidine HCl + 2H₂O, 43.9 mM mannitol, 184 mM sucrose, pH 6.0, which does not contain mAb-1.

The mAb-4 formulation consisting of mAb-4, which is a type IgG4 antibody, concentrated at 80 mg/mL and formulated with 0.400 mg/mL polysorbate 20, 10 mM acetate, 240 mM trehalose, pH 5.2.

The placebo to mAb-4 formulation is a formulation containing 0.400 g/mL polysorbate 20, 10 mM acetate, 240 mM trehalose, pH 5.2, which does not contain mAb-4.

### b) mAb Stability Studies

The stability study of mAb-1 and its corresponding placebo was performed for 3 months at storage condition of 25 °C (room temperature) in 20R glass vials (content: 20 mL). Stability studies of mAb-2 were performed for 6 months at storage condition of 25 °C (room temperature)/ 60 % RH (relative humidity) in 20R glass vials.

The stability study of mAb-4 and its corresponding placebo was performed for 6 months at storage condition of 25 °C (room temperature)/ 60% RH (relative humidity) in 6R glass vials (content: 3 mL).

### c) Sample preparation of artificially stressed polysorbate 20

Experiment for the identification of PEG-6-laurate:
1.5 mM 2,2'-azobis-2-methyl-propanimidamide, dihydrochloride (AAPH) was added to a 0.040 mg/mL aqueous polysorbate 20 solution. The polysorbate 20 solution was stressed for 40 h at 50 °C.

For correlation with the total polysorbate 20 degradation:
1.5 mM 2,2'-azobis-2-methyl-propanimidamide, dihydrochloride (AAPH) was added to a 0.040 mg/mL aqueous polysorbate 20 solution. The polysorbate 20 solution was stressed for 35h at 50 °C. After 0, 1, 3, 8, 12, 24 and 35h samples were taken from the solution and immediately frozen at -20 °C in order to stop the degradation at each time point.

### 1.2 Methods

### a) Screening of intact polysorbate 20-species via Liquid Chromatography-Mass Spectrometry (LC-MS POE)

Liquid chromatography:
The polysorbate characterization method used a reversed-phase chromatography (Kinetex XB-C C18, 2.6 µm, 2.1x150 mm, Phenomenex) operated at 50 °C with an flow of 0.250 mL/min for the separation of polysorbate species in the order of their increased hydrophobicity (1200 Series, Agilent) using water as mobile phase (MP) A and 1 % formic acid in acetonitrile (ACN) as mobile phase (MP) B. The analytical gradient started at 5 % MP B with a linear ramp to 100 % MP B at 20 min until 25 min. MP B was decreased to 5 % in a 0.1 min ramp at 25.1 min and was followed by a column wash and re-equilibration step at 5 % MP B until 30 min. Column load was usually 1-2 µg of polysorbate sample in ACN.

Mass-Spectrometry:
The separated species were then ionized with a heated electrospray ionization (HESI) source (Ion Max Ion source with HESI probe, Thermo Fisher Scientific). The instrument was operated in a positive ionization mode with source voltage of 4 kV at 310 °C. Fatty acid specific fragments generated by the following in-source fragmentation were detected in the mass spectrometer (LTQ, Thermo Fisher Scientific). The capillary temperature was at 275 °C and was operated at 43 kV. Full-scan spectra were collected over the m/z range of 100-2000 amu (atomic mass unit). Fatty acid esters of polysorbate 20 were evaluated by generating extracted ion chromatograms (EICs or XICs) out of the full scan data to get peak areas of selected compounds using LCquan^{™} (Software for quantitative analysis of liquid chromatography). Fragments of species with the same fatty acids were differed by characterized retention time.

### b) High resolution-Mass Spectrometry (HR-MS)

Liquid chromatography:
For the chromatographic conditions it is referred to the above chapter a) (LC-MS POE).

Mass-Spectrometry:
The separated species were ionized with heated electrospray ionization (HESI) source (Ion Max Ion source with HESI probe, Thermo Fisher Scientific). The instrument was operated in a positive ionization mode with source voltage of 4 kV at 310 °C. Fatty acid specific fragments generated by the following in-source fragmentation were detected in the mass spectrometer (LTQ Orbitrap XL, Thermo Fisher Scientific). The capillary temperature was at 275 °C and was operated at 9 kV. Full-scan spectra with resolution of 30000 were collected over the m/z range of 100.0-2000.0 amu. The structures were identified via accurate determination of monoisotopic peaks.

### c) Quantification of oxidation marker PEG-6-laurate (P6L) via Liquid Chromatography-Mass Spectrometry (LCMS P6L)

Liquid chromatography:
For the chromatographic conditions of the LC-MS P6L method it is referred to the above chapter a) (LC-MS POE). The column load was 1 µg of the polysorbate sample in ACN.

Mass-Spectrometry:
The separated species were then ionized with heated electrospray ionization (HESI) source (Ion Max Ion source with HESI probe, Thermo Fisher Scientific). The instrument was operated in a positive ionization mode with source voltage of 4 kV at 310 °C. Ions generated were detected in the mass spectrometer (LTQ, Thermo Fisher Scientific). The capillary temperature was at 275 °C and was operated at 43 kV. Full-scan spectra were collected over the m/z range of 300.0-550.0 amu. PEG-6-laurate was evaluated by generating extracted ion chromatograms (EIC's) out of the full scan data to get peak areas of selected compounds using LCquan^{™} (Software for quantitative analysis of liquid chromatography).

### d) Quantification of polysorbate 20 via High Performance Liquid Chromatography-Charged Aerosol Detection (HPLC-CAD)

mAb and placebo samples were diluted 1:2 with water to a polysorbate 20 concentration within the methods calibration range. An external calibration curve using aqueous polysorbate 20 solutions was prepared. Injection volume was 10 µL. HPLC-CAD was measured on a CAD Veo RS (Thermo Fisher Scientific) coupled to an Agilent 1290 LC-System (Agilent). The chromatographic separation was performed with help of mixed-mode column Oasis MAX online (20 x 2.1 mm, 30 µm, Waters) at 1 ml/mL and 30 °C column temperature. MP A was composed of 10 mM ammonium formate, 20 % 2-propanol, pH 3; MP B was composed of acetonitrile/2-propanol 50/50 % (v/v). The analytical gradient started at 0 % MP B isocratic followed by a 0.1 min ramp to 10 % MP B at 14.0 - 14.1 min. At 20 min the MP B was increased to 0 % and re-equilibrated until 25 min. The CAD was operated with 70 °C evaporation temperature, power function 1.0, filter constant 5 sec and 10 Hz data collection rate. The recorded chromatograms were integrated and the polysorbate 20 concentrations were calculated using a quadratic regression (Empower).

### e) Determination of Free Fatty Acids (FFA) via Liquid Chromatography-Mass Spectrometry (LC-MS FFA)

The polysorbate characterization method determined free fatty acids namely lauric acid, myristic acid, palmitic acid, stearic acid and oleic acid via LC-MS. mAb and polysorbate 20 samples were diluted to a final polysorbate 20 concentration of 0.040 mg/mL with water. The injection volume was 20 µL. For chromatographic separation a RP column at 50 °C (Jupiter C18, 3 µm, 2x150 mm, Phenomenex) and a HPLC system with 0.300 mL/min flow (LC-30AD, SIL-30 AC, Shimadzu) were used. 10 mM Ammonium acetate was used as MB A and 2-propanol as MB. The analytical gradient started at 50 % MP B with a linear ramp to 100 % MP B at 1 min until 2.5 min. At 3.5 min the MP B was increased to 100 % in a 0.1 min ramp. MP B was decreased to 50 % in a 0.1 min ramp at 5 min and was followed by a column wash and re-equilibration step at 50 % MP B until 11 min.

The FFA were ionized with HESI (Ion Max Ion source with HESI probe, Thermo Fisher Scientific) and analysed in the mass spectrometer (LTQ, Thermo Fisher Scientific) in negative mode. The instrument was operated in a positive ionization mode with source voltage of 3.5 kV at 310 °C. Fatty acids were detected in mass spectrometer (LTQ, Thermo Fisher Scientific). The capillary temperature was at 275 °C and was operated at -49 kV. Full-scan spectra were collected over the m/z range of 150.0-300.0 amu. Finally, extracted ion chromatograms (EIC's) were generated out of the full scan data using LCquan^{™} (Software for quantitative analysis of liquid chromatography). By using FFA calibration standards (10-1500 ng/mL) a quantitative determination was performed.

### 2. Characterization of method performance of LC-MS P6L

In order to verify whether the LC-MS based method is a realiable and suitable test method, the method was investigated in terms of linearity, specificity, accuracy, and precision according to procedures well-known in prior art.

### Linearity:

Initially, the linearity of the method was examined by evaluation of the standard curves coefficient of determination (R²). R²-evaluation of the standard curve of 1-25 ng/mL PEG-6-laurate reveals a good linearity of the method (R²=0.9979). The standard curve of LC-MS P6L method is shown in Figure 8.

Figure 8 shows a standard curve of PEG-6-laurate by plotting detected area of LC-MS analysis (LC-MS P6L) (x-axis) against the concentrations of standard solutions (1-25 ng/mL) (y-axis). The linear fit of the standard curve shows a coefficient of determination (R²) of 0.9979. Therefore, the method has a good linearity.

### Specificity:

Specificity analysis using mAb-3 and placebo to mAb-3 spiked with synthetic PEG-6-laurate revealed no interference of API, polysorbate 20 or other formulation compounds with the signal of spiked PEG-6-laurate. That is, PEG-6-laurate was detected in the spiked samples but no interfering signal was detected in the control solutions. Since PEG-6-laurate was only detected in spiked samples, the specificity of the LC-MS P6L method is given. The results are summarized in Table 1.

### Precision and accuracy:

Precision and accuracy evaluation was performed via analysis of NBE solutions spiked with 2.5 ng/nL and 20 ng/nL synthetic PEG-6-laurate in three independently prepared replicates per concentration (n=3 per PEG-6-laurate concentration). Results of the evaluation of precision show low relative standard deviations (%RSD) for both levels of PEG-6-laurate samples (%RSD at 2.5 ng/mL PEG-6-laurate: 2 %; %RSD at 20 ng/mL: 3 %). Further, the relative mean recoveries of both PEG-6-laurate samples to the target level (spike concentration) show good results varying between 90-104 % showing the good precision and accuracy of the LC-MS P6L method. The results are also summarized in Table 1.

In the following Table 1 the results of evaluation of specificity, precision and accuracy parameters of the method to detect and quantify PEG-6-laurate (P6L) are shown.

**Table 1**

| **Parameter** | **Sample** | **Conc. of P6L Spike [ng/mL]** | **Detected P6L conc. [ng/mL]** | **Relative Standard Deviation [%]** | **Recovery [%]** |
|---|---|---|---|---|---|
| **Specificity** | NBE solution | - | n.d. | - | - |
| | | 35 | 35 | | |
| | Formulation buffer without NBE | - | n.d. | | |
| | | 35 | 30 | | |
| **Precision and Accuracy** | NBE solution | 2.5 (n=3) | 2.5-2.6 | 2 | 100-104 |
| | | 20 (n=3) | 18-19 | 3 | 90-95 |

| | | | | | |
|---|---|---|---|---|---|
| n.d......not detected | | | | | |

In summary, the characterization of the LC-MS based method of the invention for quantification of PEG-6-laurate shows good performance and is capable to determine the level of PEG-6-laurate in NBE solution specific, precise and accurate. Hereafter, the method LC-MS P6L can be used for identification of critical PEG-6-laurate threshold in NBEs and thereby provide an analytical tool during formulation and process development of aqueous formulations such as biopharmaceuticals.

### References

1. Borisov OV, Ji JA, Wang YJ. Oxidative Degradation of Polysorbate Surfactants Studied by Liquid Chromatography-Mass Spectrometry. J Pharm Sci. 2015;104(3):1005-1018. doi:10.1002/jps.24314;
2. Kishore RSK, Kiese S, Fischer S, Pappenberger A, Grauschopf U, Mahler H-C. The Degradation of Polysorbates 20 and 80 and its Potential Impact on the Stability of Biotherapeutics. Pharmaceut Res. 2011;28(5):1194-1210. doi:10.1007/s11095-011-0385-x;
3. Zhang L, Yadav S, Wang YJ, Mozziconacci O, Schöneich C. Dual Effect of Histidine on Polysorbate 20 Stability: Mechanistic Studies. Pharmaceut Res. 2018; 35(2):33. doi:10.1007/s11095-017-2321-1;
4. Dahotre S, Tomlinson A, Lin B, Yadav S. Novel markers to track oxidative polysorbate degradation in pharmaceutical formulations. J Pharmaceut Biomed. 2018; 157:201-207. doi:10.1016/j.jpba.2018.05.031;
5. Kranz W, Wuchner K, Corradini E, Berger M, Hawe A. Factors Influencing Polysorbate's Sensitivity Against Enzymatic Hydrolysis and Oxidative Degradation. J Pharm Sci. 2019;108(6):2022-2032. doi:10.1016/j.xphs.2019.01.006;
6. Hewitt D, Alvarez M, Robinson K, et al. Mixed-mode and reversed-phase liquid chromatography-tandem mass spectrometry methodologies to study composition and base hydrolysis of polysorbate 20 and 80. J Chromatogr A. 2011;1218(15):2138-2145. doi:10.1016/j.chroma.2010.09.057;
7. Hama I, Okamoto T, Nakamura H. Preparation and properties of ethoxylated fatty methyl ester nonionics. J Am Oil Chem Soc. 1995;72(7):781-784. doi:10.1007/bf02541025;
8. WO 2000/038621 A1;
9. US 2002/0155962 A1;
10. O. V. Borisov, J. A. Ji, Y. J. Wang, F. Vega, and V. T. Ling. Analytical Chemistry 2011 83(10), 3934-3942. doi: 10.1021/ac2005789;
11. ICH International Conference on Harmonisation of Technical Requirements for Registration of Pharmaceuticals for Human Use: ICH Harmonised Tripartite Guideline; Validation of Analytical Procedures: Text and Methodology Q2(R1). pages 1 - 13;
12. Kromidas, Stavros, and Kuss, Hans -Joachim. Handbuch Validierung in der Analytik. Weinheim: John Wiley & Sons, Incorporated, 2009; pages: 360 - 366;
13. Zhang L. et al: "Degradation Mechanisms of Polysorbate 20 Differentiated by 18O-labeling and Mass Spectrometry", Pharmaceutical Research, Springer US, New York, Vol. 34, No.1, 13 October 2016, pp. 84-100;
14. WO 2020/174064 A1; and
15. WO 2017/117311 A1.

## Claims

1. A method for determining the degree of oxidative degradation of polysorbate 20 in an aqueous formulation containing polysorbate 20, said method comprising the following steps:
| | |
|---|---|
| step 1 | providing a first sample of the aqueous formulation to be examined, which serves as a reference sample; |
| step 2 | providing a second sample and optionally further samples of the aqueous formulation to be examined; |
| step 3 | using a liquid chromatography-mass spectrometry based method for separating polyethylene glycol-6-laurate and determining the peak area of polyethylene glycol-6-laurate, the oxidation marker for the oxidative degradation of polysorbate 20, from the reference sample of step 1, and from the second sample and each optional further sample of step 2, respectively, and determining the relative peak area of polyethylene glycol-6-laurate based on the peak area of polyethylene glycol-6-laurate in the second and each optional further sample in relation to the peak area of polyethylene glycol-6-laurate in the reference sample, the relative peak area being proportional to the degree of oxidative degradation of polysorbate 20; and |
| step 4 | optionally quantifying the amount of polyethylene glycol-6-laurate in the second sample and each optional further sample based on the relative peak area of the respective sample. |

2. The method according to claim 1,
**characterized in that**
the reference sample provided is an aqueous solution containing polysorbate 20, the polysorbate 20 content being the same as in the aqueous formulation to be examined.

3. The method according to claim 1,
**characterized in that**
in step 2, two, three or more further samples of the aqueous formulation containing polysorbate 20 are taken at successive time intervals T1, T2, T3...., which may be of equal or different length.

4. The method according to claim 1,
**characterized in that**
the relative peak area of polyethylene glycol-6-laurate for the second sample and each optional further sample in step 3 is determined by
- calculating the difference of the peak areas obtained, wherein the peak area of polyethylene glycol-6-laurate in the reference sample is subtracted, respectively, from the peak area of polyethylene glycol-6-laurate in the second sample and from the peak area of polyethylene glycol-6-laurate in each of the optional further sample(s);
or
- normalising the peak areas obtained, wherein the peak area of polyethylene glycol-6-laurate in the reference sample is set equal to 100% and the peak area of polyethylene glycol-6-laurate in the second sample and in each of the optional further sample(s) is calculated respectively based thereon.

5. A method for determining the degree of oxidative degradation of polysorbate 20 in a number of aqueous formulations each containing polysorbate 20, in particular each aqueous formulation containing the same amount of polysorbate 20, said method comprising the following steps:
| | |
|---|---|
| step 1a | providing a sample of each aqueous formulation to be examined; |
| step 2a | using a liquid chromatography and mass spectrometry based method for separating polyethylene glycol-6-laurate and determining the peak area of polyethylene glycol-6-laurate, the oxidation marker for the oxidative degradation of polysorbate 20, from each sample of step 1a, and |
| | comparing the peak areas of the samples with each other, wherein the difference in peak area of the samples is proportional to the degree of oxidative degradation of polysorbate 20 in each respective sample; and |
| step 3a | optionally quantifying the amount of polyethylene glycol-6-laurate based in each sample on the obtained peak area of the respective sample. |

6. The method according to claim 5,
**characterized in that**
the relative peak area of polyethylene glycol-6-laurate in each sample is determined in relation to a reference sample by
- calculating the difference of the peak areas obtained, wherein the peak area of polyethylene glycol-6-laurate in a reference sample is subtracted from the peak area of polyethylene glycol-6-laurate in each sample of step 2a;
or
- normalising the peak areas obtained, wherein the peak area of polyethylene glycol-6-laurate in a reference sample is set equal to 100% and the peak area of polyethylene glycol-6-laurate in each sample of step 2a is calculated based thereon.

7. The method according to any of the preceding claims 1 to 6,
**characterized in that**
one, two or more of the following conditions are fulfilled:
- a polar solvent is added to the polysorbate-containing sample to be investigated before liquid chromatography is carried out, the polar solvent being in particular the solvent used in liquid chromatography;
- the liquid chromatography is used as separation chromatography, particularly in the form of column chromatography, more particularly used as reversed-phase chromatography;
- the liquid chromatography is used as high performance liquid chromatography, wherein a gradient elution may be used;
- the liquid chromatography is used as ultra performance liquid chromatography, wherein a gradient elution may be used;
- the mass spectrometry is selected to be a high resolution-mass spectrometry, particularly for the purpose of structural elucidation;
- the polyethylene glycol-6-laurate is separated by liquid chromatography using its hydrophobic property; and/or
- the quantification in step 4 or step 3a is performed with an external calibration using standard solutions of polyethylene glycol-6-laurate.

8. The method according to any of the preceding claims 1 to 7,
**characterized in that**
for the data evaluation of the liquid chromatography-mass spectrometry based method, extracted ion chromatograms are generated from a mass-to-charge ratio which comprises a range of about 487 amu, in particular comprising or consisting of a range of 486.5 to 487.5 amu.

9. The method according to any of the preceding claims 1 to 8,
**characterized in that**
an additional step 5, which follows step 4 or step 3, or an additional step 5', which follows step 4' or step 3', or an additional step 4a which follows step 3a or step 2a, is performed each of which comprises estimating to what extent polysorbate 20 has already been oxidatively degraded in the sample based on an inversely proportional relationship between the polyethylene glycol-6-laurate level and the polysorbate 20 level.

10. The method according to any of the preceding claims 1 to 9,
**characterized in that**
an aqueous biopharmaceutical formulation is used as the aqueous formulation.

11. The method according to any of the preceding claims 1 to 10,
**characterized in that**
the polysorbate 20 present in a sample is
- intended for pharmaceutical use, particularly for use in drugs; and/or
- suitable for parenteral administration.

12. The method according to any of the preceding claims 1 to 11,
**characterized in that**
the aqueous formulation contains one or more proteins, wherein the protein(s) may be selected from a monoclonal antibody, polyclonal antibody, monospecific antibody, multispecific antibody, single chain antibodies or antibody fragments thereof, such as, Fv, scFv, Fab, Fab', scFab, F(ab')2, Fab2, Fc and Fc'-fragments, heavy and light immunoglobulin chains and their constant, variable or hypervariable region as well as Fv- and Fd-fragments, diabody, triabody, scFv-Fc, minibody, or single domain antibody or a mixture thereof,
wherein the protein(s) may be selected from therapeutic protein(s), which are used for the prevention or treatment of a disease or a disorder.

13. The method according to any of the preceding claims 1 to 12,
**characterized in that**
the method is performed as a stability testing during the development, manufacture, shelf life, or storage period of an aqueous formulation or a number of aqueous formulations that respectively contain polysorbate 20 for determining the degree of oxidative degradation of polysorbate 20 in the aqueous formulation(s).

14. A process of producing a recombinant protein, said process comprising the following steps:
| | |
|---|---|
| step a) | cultivating in cell culture an eukaryotic cell expressing a recombinant protein in cell culture; |
| step b) | harvesting the recombinant protein; |
| step c) | purifying the recombinant protein using an aqueous formulation; |
| step d) | using polysorbate 20, formulating the recombinant protein into a pharmaceutically acceptable aqueous formulation suitable for administration; and |
| step e) | obtaining at least one sample of the pharmaceutically acceptable aqueous formulation of step d); |
wherein the process additionally comprises performing a method for detecting the degree of oxidative degradation of polysorbate 20 according to any of the preceding claims 1 to 13 with the sample obtained in step e), wherein the sample from step d) may be a drug substance sample or a drug product sample.

15. Use of polyethylene glycol-6-laurate as oxidation marker in a method for determining the degree of oxidative degradation of polysorbate 20 in an aqueous formulation or a number of aqueous formulations that respectively contain polysorbate 20, whereby the method is according to any of the preceding claims 1 to 13.

## Patentansprüche

1. Verfahren zur Bestimmung des Grades des oxidativen Abbaus von Polysorbat 20 in einer wässrigen Formulierung, die Polysorbat 20 enthält, wobei das Verfahren die folgenden Schritte umfasst:
| | |
|---|---|
| Schritt 1 | Bereitstellen einer ersten Probe der zu untersuchenden wässrigen Formulierung, die als Referenzprobe dient; |
| Schritt 2 | Bereitstellen einer zweiten Probe und gegebenenfalls weiterer Proben der zu untersuchenden wässrigen Formulierung; |
| Schritt 3 | Verwenden eines auf Flüssigchromatographie-Massenspektrometrie basierenden Verfahrens zum Abtrennen von Polyethylenglykol-6-laurat und zum Bestimmen der Peakfläche von Polyethylenglykol-6-laurat, dem Oxidationsmarker für den oxidativen Abbau von Polysorbat 20, aus der Referenzprobe von Schritt 1 sowie aus der zweiten Probe und jeder optionalen weiteren Probe von Schritt 2, und Bestimmen der relativen Peakfläche von Polyethylenglykol-6-laurat, basierend auf der Peakfläche von Polyethylenglykol-6-laurat, in der zweiten und jeder optionalen weiteren Probe im Verhältnis zur Peakfläche von Polyethylenglykol-6-laurat in der Referenzprobe, wobei die relative Peakfläche proportional zum Grad des oxidativen Abbaus von Polysorbat 20 ist; und |
| Schritt 4 | gegebenenfalls Quantifizierung der Menge an Polyethylenglykol-6-laurat in der zweiten Probe und jeder optionalen weiteren Probe, basierend auf der relativen Peakfläche der jeweiligen Probe. |

2. Verfahren nach Anspruch 1,
**dadurch gekennzeichnet, dass**
die bereitgestellte Referenzprobe eine wässrige Lösung ist, die Polysorbat 20 enthält, wobei der Gehalt an Polysorbat 20 dem in der zu untersuchenden wässrigen Formulierung entspricht.

3. Verfahren nach Anspruch 1,
**dadurch gekennzeichnet, dass**
in Schritt 2 zwei, drei oder mehr weitere Proben der wässrigen Formulierung, die Polysorbat 20 enthalten, in aufeinanderfolgenden Zeitintervallen T1, T2, T3... entnommen werden, die gleich oder unterschiedlich lang sein können.

4. Verfahren nach Anspruch 1,
**dadurch gekennzeichnet, dass**
die relative Peakfläche von Polyethylenglykol-6-laurat für die zweite Probe und jede optionale weitere Probe in Schritt 3 bestimmt wird durch
- Berechnen der Differenz der erhaltenen Peakflächen, wobei die Peakfläche von Polyethylenglykol-6-laurat in der Referenzprobe jeweils von der Peakfläche von Polyethylenglykol-6-laurat in der zweiten Probe und von der Peakfläche von Polyethylenglykol-6-laurat in jeder der optionalen weiteren Probe(n) subtrahiert wird;
oder
- durch Normalisieren der erhaltenen Peakflächen, wobei die Peakfläche von Polyethylenglykol-6-laurat in der Referenzprobe auf 100 % gesetzt wird und die Peakfläche von Polyethylenglykol-6-laurat in der zweiten Probe sowie in jeder der optionalen weiteren Probe(n) jeweils darauf basierend berechnet wird.

5. Verfahren zur Bestimmung des Grades des oxidativen Abbaus von Polysorbat 20 in einer Anzahl von wässrigen Formulierungen, die jeweils Polysorbat 20 enthalten, insbesondere wobei jede wässrige Formulierung die gleiche Menge an Polysorbat 20 enthält, wobei das Verfahren die folgenden Schritte umfasst:
| | |
|---|---|
| Schritt 1a | Bereitstellen einer Probe jeder zu untersuchenden wässrigen Formulierung; |
| Schritt 2a | Verwenden eines auf Flüssigchromatographie und Massenspektrometrie basierenden Verfahrens zum Abtrennen von Polyethylenglykol-6-laurat und zum Bestimmen der Peakfläche von Polyethylenglykol-6-laurat, dem Oxidationsmarker für den oxidativen Abbau von Polysorbat 20, aus jeder Probe von Schritt 1a, und |
| | Vergleichen der Peakflächen der Proben miteinander, wobei die Differenz der Peakflächen der Proben proportional zum Grad des oxidativen Abbaus von Polysorbat 20 in der jeweiligen Probe ist; und |
| Schritt 3a | gegebenenfalls Quantifizieren der Menge an Polyethylenglykol-6-laurat in jeder Probe, basierend auf der erhaltenen Peakfläche der jeweiligen Probe. |

6. Verfahren nach Anspruch 5,
**dadurch gekennzeichnet, dass**
die relative Peakfläche von Polyethylenglykol-6-laurat in jeder Probe im Verhältnis zu einer Referenzprobe bestimmt wird durch
- Berechnen der Differenz der erhaltenen Peakflächen, wobei die Peakfläche von Polyethylenglykol-6-laurat in einer Referenzprobe von der Peakfläche von Polyethylenglykol-6-laurat in jeder Probe von Schritt 2a subtrahiert wird;
oder
- Normalisieren der erhaltenen Peakflächen, wobei die Peakfläche von Polyethylenglykol-6-laurat in einer Referenzprobe auf 100 % gesetzt wird und die Peakfläche von Polyethylenglykol-6-laurat in jeder Probe von Schritt 2a darauf basierend berechnet wird.

7. Verfahren nach irgendeinem der vorstehenden Ansprüche 1 bis 6,
**dadurch gekennzeichnet, dass**
eine, zwei oder mehrere der folgenden Bedingungen erfüllt sind:
- der zu untersuchenden, Polysorbat enthaltenden Probe wird vor der Durchführung der Flüssigchromatographie ein polares Lösungsmittel zugesetzt, wobei das polare Lösungsmittel insbesondere das in der Flüssigchromatographie verwendete Lösungsmittel ist;
- die Flüssigchromatographie wird als Trennungschromatographie eingesetzt, insbesondere in Form von Säulenchromatographie, insbesondere als Umkehrphasenchromatographie;
- die Flüssigchromatographie wird als Hochleistungsflüssigchromatographie eingesetzt, wobei eine Gradientenelution verwendet werden kann;
- die Flüssigchromatographie wird als Ultra-Hochleistungsflüssigchromatographie eingesetzt, wobei eine Gradientenelution verwendet werden kann;
- die Massenspektrometrie wird als hochauflösende Massenspektrometrie ausgewählt, insbesondere zum Zweck der Strukturaufklärung;
- das Polyethylenglykol-6-laurat wird mittels Flüssigchromatographie unter Ausnutzung seiner hydrophoben Eigenschaft abgetrennt; und/oder
- die Quantifizierung in Schritt 4 oder Schritt 3a erfolgt mittels einer externen Kalibrierung unter Verwendung von Standardlösungen von Polyethylenglykol-6-laurat.

8. Verfahren nach irgendeinem der vorstehenden Ansprüche 1 bis 7,
**dadurch gekennzeichnet, dass**
für die Datenauswertung des auf Flüssigchromatographie-Massenspektrometrie basierenden Verfahrens extrahierte Ionenchromatogramme aus einem Masse-zu-Ladungs-Verhältnis erzeugt werden, das einen Bereich von etwa 487 amu, insbesondere einen Bereich von 486,5 bis 487,5 amu umfasst oder daraus besteht.

9. Verfahren nach irgendeinem der vorstehenden Ansprüche 1 bis 8,
**dadurch gekennzeichnet, dass**
ein zusätzlicher Schritt 5, der auf Schritt 4 oder Schritt 3 folgt, oder ein zusätzlicher Schritt 5', der auf Schritt 4' oder Schritt 3' folgt, oder ein zusätzlicher Schritt 4a, der auf Schritt 3a oder Schritt 2a folgt, durchgeführt wird, wobei jeder dieser Schritte das Abschätzen umfasst, inwieweit Polysorbat 20 in der Probe bereits oxidativ abgebaut wurde, basierend auf einer umgekehrt proportionalen Beziehung zwischen dem Polyethylenglykol-6-laurat-Gehalt und dem Polysorbat-20-Gehalt.

10. Verfahren nach irgendeinem der vorstehenden Ansprüche 1 bis 9,
**dadurch gekennzeichnet, dass**
eine wässrige biopharmazeutische Formulierung als wässrige Formulierung verwendet wird.

11. Verfahren nach irgendeinem der vorstehenden Ansprüche 1 bis 10,
**dadurch gekennzeichnet, dass**
das in einer Probe vorhandene Polysorbat 20
- für pharmazeutische Zwecke, insbesondere zur Verwendung in Arzneimitteln, bestimmt ist; und/oder
- für die parenterale Verabreichung geeignet ist.

12. Verfahren nach irgendeinem der vorstehenden Ansprüche 1 bis 11,
**dadurch gekennzeichnet, dass**
die wässrige Formulierung ein oder mehrere Proteine enthält, wobei das Protein bzw. die Proteine ausgewählt werden aus einem monoklonalen Antikörper, einem polyklonalen Antikörper, einem monospezifischen Antikörper, einem multispezifischen Antikörper, Einzelketten-Antikörpern oder Antikörperfragmenten davon, wie beispielsweise Fv-, scFv-, Fab-, Fab'-, scFab-, F(ab')2-, Fab2-, Fc- und Fc'-Fragmente, schwere und leichte Immunglobulinketten und deren konstante, variable oder hypervariable Region sowie Fv- und Fd-Fragmente, Diabody, Triabody, scFv-Fc, Minibody oder Einzeldomänen-Antikörper oder eine Mischung davon, wobei das Protein bzw. die Proteine aus therapeutischen Proteinen ausgewählt sein können, die zur Vorbeugung oder Behandlung einer Krankheit oder einer Störung verwendet werden.

13. Verfahren nach irgendeinem der vorstehenden Ansprüche 1 bis 12,
**dadurch gekennzeichnet, dass**
das Verfahren als Stabilitätstest während der Entwicklung, Herstellung, Haltbarkeitsdauer oder Lagerzeit einer wässrigen Formulierung oder einer Anzahl von wässrigen Formulierungen durchgeführt wird, die jeweils Polysorbat 20 enthalten, um den Grad des oxidativen Abbaus von Polysorbat 20 in der/den wässrigen Formulierung(en) zu bestimmen.

14. Verfahren zur Herstellung eines rekombinanten Proteins, wobei das Verfahren die folgenden Schritte umfasst:
| | |
|---|---|
| Schritt a) | Kultivieren einer eukaryotischen Zelle, die ein rekombinantes Protein exprimiert, in einer Zellkultur; |
| Schritt b) | Gewinnen des rekombinanten Proteins; |
| Schritt c) | Reinigen des rekombinanten Proteins unter Verwendung einer wässrigen Formulierung; |
| Schritt d) | Formulieren des rekombinanten Proteins zu einer pharmazeutisch akzeptablen, zur Verabreichung geeigneten wässrigen Formulierung unter Verwendung von Polysorbat 20; und |
| Schritt e) | Erhalten mindestens einer Probe der pharmazeutisch akzeptablen wässrigen Formulierung von Schritt d); |
wobei das Verfahren zusätzlich die Durchführung eines Verfahrens zum Bestimmen des Grades des oxidativen Abbaus von Polysorbat 20 gemäß einem der vorstehenden Ansprüche 1 bis 13 mit der in Schritt e) erhaltenen Probe umfasst, wobei die Probe von Schritt d) eine Wirkstoffprobe oder eine Arzneimittelprobe sein kann.

15. Verwendung von Polyethylenglykol-6-laurat als Oxidationsmarker in einem Verfahren zum Bestimmen des Grades des oxidativen Abbaus von Polysorbat 20 in einer wässrigen Formulierung oder einer Anzahl von wässrigen Formulierungen, die jeweils Polysorbat 20 enthalten, wobei das Verfahren gemäß irgendeinem der vorstehenden Ansprüche 1 bis 13 ist.

## Revendications

1. Méthode de détermination du degré de dégradation oxydative du polysorbate 20 dans une formulation aqueuse contenant du polysorbate 20, ladite méthode comprenant les étapes suivantes :
une étape 1 de fourniture d'un premier échantillon de la formulation aqueuse à examiner, qui sert d'échantillon de référence ;
une étape 2 de fourniture d'un deuxième échantillon et facultativement d'échantillons supplémentaires de la formulation aqueuse à examiner ;
une étape 3 d'utilisation d'une méthode basée sur la chromatographie liquide-spectrométrie de masse pour séparer le polyéthylène glycol-6-laurate et déterminer la surface du pic du polyéthylène glycol-6-laurate, le marqueur d'oxydation pour la dégradation oxydative du polysorbate 20, à partir de l'échantillon de référence de l'étape 1, et à partir du deuxième échantillon et de chaque échantillon supplémentaire facultatif de l'étape 2, respectivement, et
de détermination de la surface relative du pic du polyéthylène glycol-6-laurate sur la base de la surface du pic du polyéthylène glycol-6-laurate dans le deuxième échantillon et chaque échantillon supplémentaire facultatif par rapport à la surface du pic du polyéthylène glycol-6-laurate dans l'échantillon de référence, la surface relative du pic étant proportionnelle au degré de dégradation oxydative du polysorbate 20 ; et
une étape 4 de quantification facultative de la quantité de polyéthylène glycol-6-laurate dans le deuxième échantillon et chaque échantillon supplémentaire facultatif sur la base de la surface relative du pic de l'échantillon respectif.

2. Méthode selon la revendication 1,
**caractérisée en ce que**
l'échantillon de référence fourni est une solution aqueuse contenant du polysorbate 20, la teneur en polysorbate 20 étant identique à celle dans la formulation aqueuse à examiner.

3. Méthode selon la revendication 1,
**caractérisée en ce que**
à l'étape 2, deux, trois ou plus de trois échantillons supplémentaires de la formulation aqueuse contenant du polysorbate 20 sont prélevés à des intervalles de temps T1, T2, T3 .... successifs, lesquels peuvent être de longueur égale ou différente.

4. Méthode selon la revendication 1,
**caractérisée en ce que**
la surface relative du pic du polyéthylène glycol-6-laurate pour le deuxième échantillon et chaque échantillon supplémentaire facultatif à l'étape 3 est déterminée par
- le calcul de la différence des surfaces de pic obtenues, dans laquelle la surface du pic du polyéthylène glycol-6-laurate dans l'échantillon de référence est soustraite, respectivement, de la surface du pic du polyéthylène glycol-6-laurate dans le deuxième échantillon et de la surface du pic du polyéthylène glycol-6-laurate dans chacun du ou des autres échantillons facultatifs ;
ou
- la normalisation des surfaces de pic obtenues, dans laquelle la surface du pic du polyéthylène glycol-6-laurate dans l'échantillon de référence est fixée à 100 % et la surface du pic du polyéthylène glycol-6-laurate dans le deuxième échantillon et dans chacun du ou des échantillons supplémentaires facultatifs est calculée respectivement sur la base de celle-ci.

5. Méthode de détermination du degré de dégradation oxydative du polysorbate 20 dans un certain nombre de formulations aqueuses contenant chacune du polysorbate 20, en particulier chaque formulation aqueuse contenant la même quantité de polysorbate 20, ladite méthode comprenant les étapes suivantes :
une étape 1a de fourniture d'un échantillon de chaque formulation aqueuse à examiner ;
une étape 2a d'utilisation d'une méthode basée sur la chromatographie liquide et la spectrométrie de masse pour séparer le polyéthylène glycol-6-laurate et déterminer la surface du pic du polyéthylène glycol-6-laurate, le marqueur d'oxydation pour la dégradation oxydative du polysorbate 20, à partir de chaque échantillon de l'étape 1a, et
la comparaison des surfaces de pic des échantillons les unes aux autres, dans laquelle la différence de surface de pic des échantillons est proportionnelle au degré de dégradation oxydative du polysorbate 20 dans chaque échantillon respectif ; et
une étape 3a de quantification facultative de la quantité de polyéthylène glycol-6-laurate sur la base, dans chaque échantillon, de la surface de pic obtenue de l'échantillon respectif.

6. Méthode selon la revendication 5,
**caractérisée en ce que**
la surface relative du pic du polyéthylène glycol-6-laurate dans chaque échantillon est déterminée par rapport à un échantillon de référence par
- le calcul de la différence des surfaces de pic obtenues, dans laquelle la surface du pic du polyéthylène glycol-6-laurate dans un échantillon de référence est soustraite de la surface du pic du polyéthylène glycol-6-laurate dans chaque échantillon de l'étape 2a ;
ou
- la normalisation des surfaces de pic obtenues, dans laquelle la surface du pic du polyéthylène glycol-6-laurate dans un échantillon de référence est fixée à 100 % et la surface du pic du polyéthylène glycol-6-laurate dans chaque échantillon de l'étape 2a est calculée sur la base de celle-ci.

7. Méthode selon l'une quelconque des revendications 1 à 6 précédentes,
**caractérisée en ce que**
une, deux ou plus de deux des conditions suivantes sont satisfaites :
- un solvant polaire est ajouté à l'échantillon contenant du polysorbate à étudier avant la réalisation de la chromatographie liquide, le solvant polaire étant en particulier le solvant utilisé dans la chromatographie liquide ;
- la chromatographie liquide utilisée est une chromatographie de séparation, en particulier sous la forme d'une chromatographie sur colonne, plus particulièrement une chromatographie en phase inversée ;
- la chromatographie liquide utilisée est une chromatographie liquide haute performance, dans laquelle une élution par gradient peut être utilisée ;
- la chromatographie liquide utilisée est une chromatographie liquide ultra-performante, dans laquelle une élution par gradient peut être utilisée ;
- la spectrométrie de masse est sélectionnée de manière à être une spectrométrie de masse à haute résolution, en particulier à des fins d'élucidation de structure ;
- le polyéthylène glycol-6-laurate est séparé par chromatographie liquide en utilisant sa propriété hydrophobe ; et/ou
- la quantification à l'étape 4 ou à l'étape 3a est réalisée avec un étalonnage externe à l'aide de solutions étalons de polyéthylène glycol-6-laurate.

8. Méthode selon l'une quelconque des revendications 1 à 7 précédentes,
**caractérisée en ce que**
pour l'évaluation des données de la méthode basée sur la chromatographie liquide-spectrométrie de masse, des chromatogrammes d'ions extraits sont générés à partir d'un rapport masse/charge qui comprend une plage d'environ 487 uma, en particulier comprenant ou consistant en une plage de 486,5 à 487,5 uma.

9. Méthode selon l'une quelconque des revendications 1 à 8 précédentes,
**caractérisée en ce que**
une étape 5 supplémentaire, qui suit l'étape 4 ou l'étape 3, ou une étape 5' supplémentaire, qui suit l'étape 4' ou l'étape 3', ou une étape 4a supplémentaire qui suit l'étape 3a ou l'étape 2a, est réalisée, chacune comprenant l'estimation de la mesure dans laquelle le polysorbate 20 a déjà été dégradé de manière oxydative dans l'échantillon sur la base d'une relation inversement proportionnelle entre le taux de polyéthylène glycol-6-laurate et le taux de polysorbate 20.

10. Méthode selon l'une quelconque des revendications 1 à 9 précédentes,
**caractérisée en ce que**
une formulation biopharmaceutique aqueuse est utilisée comme formulation aqueuse.

11. Méthode selon l'une quelconque des revendications 1 à 10 précédentes,
**caractérisée en ce que**
le polysorbate 20 présent dans un échantillon est
- destiné à un usage pharmaceutique, en particulier à une utilisation dans des médicaments ; et/ou
- approprié pour une administration parentérale.

12. Méthode selon l'une quelconque des revendications 1 à 11 précédentes,
**caractérisée en ce que**
la formulation aqueuse contient une ou plusieurs protéines, dans laquelle la ou les protéines peuvent être sélectionnées parmi un anticorps monoclonal, un anticorps polyclonal, un anticorps monospécifique, un anticorps multispécifique, des anticorps à chaîne unique ou des fragments d'anticorps de ceux-ci, tels que les fragments Fv, scFv, Fab, Fab', scFab, F(ab')2, Fab2, Fc et Fc', les chaînes lourdes et légères d'immunoglobulines et leur région constante, variable ou hypervariable, ainsi que les fragments Fv et Fd, un diabody, un tribody, un scFv-Fc, un minibody, ou un anticorps à domaine unique ou un mélange de ceux-ci,
dans laquelle la ou les protéines peuvent être sélectionnées parmi une ou plusieurs protéines thérapeutiques, lesquelles sont utilisées pour la prévention ou le traitement d'une maladie ou d'un trouble.

13. Méthode selon l'une quelconque des revendications 1 à 12 précédentes,
**caractérisée en ce que**
la méthode est réalisée en tant que test de stabilité pendant le développement, la fabrication, la durée de conservation ou la période de stockage d'une formulation aqueuse ou d'un certain nombre de formulations aqueuses qui contiennent respectivement du polysorbate 20, afin de déterminer le degré de dégradation oxydative du polysorbate 20 dans la ou les formulations aqueuses.

14. Procédé de production d'une protéine recombinante, ledit procédé comprenant les étapes suivantes :
une étape a) de mise en culture cellulaire d'une cellule eucaryote exprimant une protéine recombinante en culture cellulaire ;
une étape b) de collecte de la protéine recombinante ;
une étape c) de purification de la protéine recombinante à l'aide d'une formulation aqueuse ;
une étape d) d'utilisation de polysorbate 20, pour formuler la protéine recombinante dans une formulation aqueuse pharmaceutiquement acceptable appropriée pour une administration ; et
une étape e) d'obtention d'au moins un échantillon de la formulation aqueuse pharmaceutiquement acceptable de l'étape d) ;
dans lequel le procédé comprend en outre la réalisation d'une méthode de détection du degré de dégradation oxydative du polysorbate 20 selon l'une quelconque des revendications 1 à 13 précédentes avec l'échantillon obtenu à l'étape e), dans lequel l'échantillon de l'étape d) peut être un échantillon de substance médicamenteuse ou un échantillon de produit médicamenteux.

15. Utilisation du polyéthylène glycol-6-laurate comme marqueur d'oxydation dans une méthode de détermination du degré de dégradation oxydative du polysorbate 20 dans une formulation aqueuse ou un certain nombre de formulations aqueuses qui contiennent respectivement du polysorbate 20, dans laquelle la méthode est selon l'une quelconque des revendications 1 à 13 précédentes.
